(19) Europäisches Patentamt
European Patent Office
Office européen des brevets

(11) EP 3 901 148 B1

(12) **EUROPEAN PATENT SPECIFICATION**

(45) Date of publication and mention of the grant of the patent:
**25.02.2026 Bulletin 2026/09**

(21) Application number: **19900999.4**

(22) Date of filing: **22.11.2019**

(51) International Patent Classification (IPC):
***C07D 403/14*** $^{(2006.01)}$ ***C07F 7/08*** $^{(2006.01)}$

(52) Cooperative Patent Classification (CPC):
**C07F 7/0814; C07D 403/14; H10K 85/654; H10K 85/6572;** H10K 50/11; H10K 85/342; H10K 2101/10; H10K 2101/90; Y02E 10/549

(86) International application number:
**PCT/KR2019/016164**

(87) International publication number:
**WO 2020/130381 (25.06.2020 Gazette 2020/26)**

(54) **COMPOUND FOR ORGANIC OPTOELECTRONIC ELEMENT, COMPOSITION FOR ORGANIC OPTOELECTRONIC ELEMENT AND ORGANIC OPTOELECTRONIC ELEMENT AND DISPLAY DEVICE**

VERBINDUNG FÜR ORGANISCHES OPTOELEKTRONISCHES ELEMENT, ZUSAMMENSETZUNG FÜR ORGANISCHES OPTOELEKTRONISCHES ELEMENT UND ORGANISCHES OPTOELEKTRONISCHES ELEMENT UND ANZEIGEVORRICHTUNG

COMPOSÉ POUR ÉLÉMENT OPTOÉLECTRONIQUE ORGANIQUE, COMPOSITION POUR ÉLÉMENT OPTOÉLECTRONIQUE ORGANIQUE ET ÉLÉMENT OPTOÉLECTRONIQUE ORGANIQUE ET DISPOSITIF D'AFFICHAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018 KR 20180167505**

(43) Date of publication of application:
**27.10.2021 Bulletin 2021/43**

(73) Proprietor: **Samsung SDI Co., Ltd.**
**Yongin-si, Gyeonggi-do 17084 (KR)**

(72) Inventors:
- **SHIN, Chang Ju**
  **Suwon-si, Gyeonggi-do 16678 (KR)**
- **KIM, Changwoo**
  **Suwon-si, Gyeonggi-do 16678 (KR)**
- **KIM, Hyung Sun**
  **Suwon-si, Gyeonggi-do 16678 (KR)**
- **RYU, Dong Wan**
  **Suwon-si, Gyeonggi-do 16678 (KR)**
- **LEE, Seungjae**
  **Suwon-si, Gyeonggi-do 16678 (KR)**
- **LEE, Hanill**
  **Suwon-si, Gyeonggi-do 16678 (KR)**
- **JUNG, Ho Kuk**
  **Suwon-si, Gyeonggi-do 16678 (KR)**
- **LUI, Jinhyun**
  **Suwon-si, Gyeonggi-do 16678 (KR)**
- **PARK, Seungin**
  **Suwon-si, Gyeonggi-do 16678 (KR)**
- **RYU, Dongkyu**
  **Suwon-si, Gyeonggi-do 16678 (KR)**
- **JUNG, Sung-Hyun**
  **Suwon-si, Gyeonggi-do 16678 (KR)**

(74) Representative: **Michalski Hüttermann & Partner Patentanwälte mbB**
**Kaistraße 16A**
**40221 Düsseldorf (DE)**

(56) References cited:

| | |
|---|---|
| **EP-A1- 3 675 194** | **EP-A1- 3 689 867** |
| **EP-A1- 3 885 345** | **WO-A1-2020/091521** |
| **WO-A1-2020/157204** | **CN-A- 103 435 597** |
| **CN-A- 103 435 597** | **CN-A- 106 883 215** |
| **CN-A- 111 233 846** | **CN-A- 112 300 152** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

KR-A- 20120 116 282 KR-A- 20160 116 297
KR-A- 20180 131 180

- DANIEL WAGNER ET AL: "Triazine Based Bipolar Host Materials for Blue Phosphorescent OLEDs", CHEMISTRY OF MATERIALS, vol. 25, no. 18, 13 August 2013 (2013-08-13), pages 3758 - 3765, XP055122413, ISSN: 0897-4756, DOI: 10.1021/cm4023216

## Description

### Technical Field

A compound for an organic optoelectronic device, a composition for an organic optoelectronic device, an organic optoelectronic device, and a display device are disclosed.

### Background Art

An organic optoelectronic device (organic optoelectronic diode) is a device that converts electrical energy into photoenergy, and vice versa.

CN 103 435 597 A refers to a 1,3,5-triazine derivative that can emit high-efficiency blue fluorescence.

Daniel Wagner ET AL: "Triazine Based Bipolar Hast Materials for Blue Phosphorescent OLEDs", Chemistry of materials, vol. 25, no. 18, 13 August 2013 (2013-08-13), pages 3758-3765, XP055122413, ISSN: 0897-4756, 001: 10.1021/cm4023216.

An organic optoelectronic device may be classified as follows in accordance with its driving principles. One is a photoelectric device where excitons are generated by photoenergy, separated into electrons and holes, and are transferred to different electrodes to generate electrical energy, and the other is a light emitting device where a voltage or a current is supplied to an electrode to generate photoenergy from electrical energy.

Examples of the organic optoelectronic device may be an organic photoelectric device, an organic light emitting diode, an organic solar cell, and an organic photo conductor drum.

Of these, an organic light emitting diode (OLED) has recently drawn attention due to an increase in demand for flat panel displays. The organic light emitting diode converts electrical energy into light, and the performance of organic light emitting diode is greatly influenced by the organic materials disposed between electrodes.

### Disclosure

### Technical Problem

An embodiment provides a compound for an organic optoelectronic device capable of realizing a high efficiency and long life-span organic optoelectronic device.

Another embodiment provides a composition for an organic optoelectronic device including the compound for an organic optoelectronic device.

Another embodiment provides an organic optoelectronic device including the compound for the organic optoelectronic device or the composition for the organic optoelectronic device.

Another embodiment provides a display device including the organic optoelectronic device.

### Technical Solution

According to an embodiment, a compound for an organic optoelectronic device represented by Chemical Formula 1 is provided.

[Chemical Formula 1]

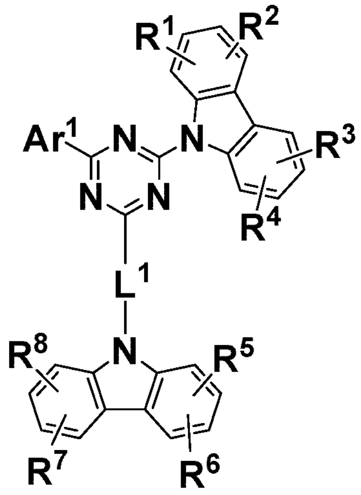

In Chemical Formula 1,

$Ar^1$ is a substituted or unsubstituted phenyl group or a substituted or unsubstituted biphenyl group,
$L^1$ is an unsubstituted biphenylene group, and
$R^1$ to $R^8$ are independently hydrogen or deuterium.

According to another embodiment, a composition for an organic optoelectronic device includes a first compound for an organic optoelectronic device and a second compound for an organic optoelectronic device, wherein the first compound for the organic optoelectronic device is represented by the Chemical Formula 1, the second compound for the organic optoelectronic device is represented by Chemical Formula 2; or a combination of Chemical Formula 3 and Chemical Formula 4.

[Chemical Formula 2]

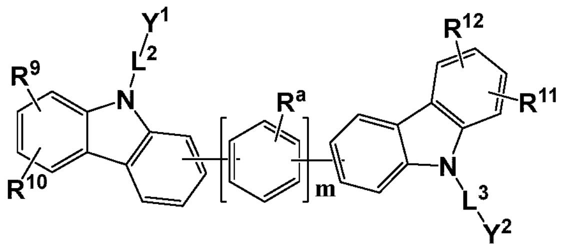

In Chemical Formula 2,

$Y^1$ and $Y^2$ are independently a substituted or unsubstituted C6 to C20 aryl group or a substituted or unsubstituted C2 to C30 heterocyclic group,
$L^2$ and $L^3$ are independently a single bond, or a substituted or unsubstituted C6 to C20 arylene group,
$R^a$ and $R^9$ to $R^{12}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, or a substituted or unsubstituted C2 to C30 heterocyclic group, and
m is an integer of 0 to 2;

[Chemical Formula 3] [Chemical Formula 4]

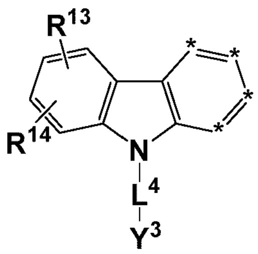

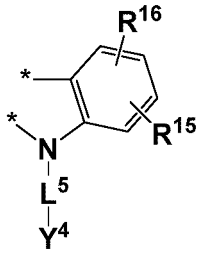

In Chemical Formula 3 and Chemical Formula 4,

$Y^3$ and $Y^4$ are independently a substituted or unsubstituted C6 to C20 aryl group or a substituted or unsubstituted C2 to C30 heterocyclic group,
adjacent two *'s of Chemical Formula 3 is linked with Chemical Formula 4,
* of Chemical Formula 3 that are not linked with Chemical Formula 4 are independently C-$L^a$-$R^b$,
$L^a$, $L^4$, and $L^5$ are independently a single bond, or a substituted or unsubstituted C6 to C20 arylene group, and
$R^b$ and $R^{13}$ to $R^{16}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

According to another embodiment, an organic optoelectronic device includes an anode and a cathode facing each other, and at least one organic layer disposed between the anode and the cathode, wherein the organic layer includes the aforementioned compound for an organic optoelectronic device or the composition for an organic optoelectronic device.

According to another embodiment, a display device including the organic optoelectronic device is provided.

**Advantageous Effects**

An organic optoelectronic device having high efficiency and a long life-span may be realized.

**Description of the Drawings**

FIGS. 1 and 2 are cross-sectional views showing organic light emitting diodes according to embodiments.

**<Description of Symbols>**

100, 200: organic light emitting diode
105: organic layer
110: cathode
120: anode
130: light emitting layer
140: hole auxiliary layer

**Best Mode**

Hereinafter, embodiments of the present invention are described in detail. However, these embodiments are exemplary, the present invention is not limited thereto and the present invention is defined by the scope of claims.

In the present specification, when a definition is not otherwise provided, "substituted" refers to replacement of at least one hydrogen of a substituent or a compound by deuterium (D), a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, or a cyano group. In addition, in specific examples of the present invention, "substituted" refers to replacement of at least one hydrogen of a substituent or a compound by deuterium, a C1 to C20 alkyl group, a C6 to C30 aryl group, or a cyano group. In addition, in specific examples of the present invention, "substituted" refers to replacement of at least one hydrogen of a substituent or a compound by deuterium, a C1 to C5 alkyl group, a C6 to C18 aryl group, a cyano group, or a C1 to C5 alkylsilyl group. In addition, in specific examples of the present invention, "substituted" refers to replacement of at least one hydrogen of a substituent or a compound by deuterium, a cyano group, a trimethylsilyl group, a phenyl group, a biphenyl group, terphenyl group, or a naphthyl group.

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and may include a group in which all elements of the hydrocarbon aromatic moiety have p-orbitals which form conjugation, for example a phenyl group, a naphthyl group, and the like, a group in which two or more hydrocarbon aromatic moieties may be linked by a sigma bond, for example a biphenyl group, a terphenyl group, a quarterphenyl group, and the like, and a group in which two or more hydrocarbon aromatic moieties are fused directly or indirectly to provide a non-aromatic fused ring, for example a fluorenyl group, and the like.

The aryl group may include a monocyclic, polycyclic or fused ring polycyclic (i.e., rings sharing adjacent pairs of carbon atoms) functional group.

In the present specification, "heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" refers to an aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, a substituted or unsubstituted dibenzothiophenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound according to an embodiment is described.

The compound according to an embodiment is represented by Chemical Formula 1.

[Chemical Formula 1]

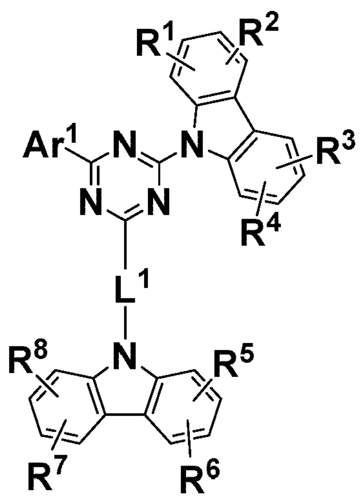

In Chemical Formula 1,

$Ar^1$ is a phenyl group or a substituted or unsubstituted biphenyl group,
$L^1$ is an unsubstituted biphenylene group,
$R^1$ to $R^8$ are independently hydrogen or deuterium.

The compound represented by Chemical Formula 1 may have a structure of one carbazole group directly linking with triazine without a linking group in an N-direction and another carbazole group linking with the triazine through biphenylene in an N-direction with the triazine in the core.

Since one carbazole group is directly linked with the triazine without a linking group in the N-direction, that is, at a position No. 9, the compound may have a relatively deep LUMO energy level and thus an advantage for electron injection and transportation.

In addition, since another carbazole group is linked with the triazine in the N-direction, that is, at the position No. 9, a π - bond through a C-N bond is broken, and thus an electron cloud between HOMO-LUMO may be clearly localized into a hole transport moiety and an electron transport moiety, and this localization may more effectively occur, as the carbazole group is linked with the triazine through the biphenylene, and resultantly, a life-span improvement effect may be maximized.

In other words, in a device including the compound represented by Chemical Formula 1, hole/electron injection and transportation may be promoted, and the electron cloud may be effectively localized, and thus a stable structure for both electrons and holes is realized, and accordingly, more advantageous characteristics for a life-span may be obtained.

Particularly, the compound includes the triazine as a central core and thus may secure rapid electron injection and mobility and have a charge balance with the carbazole moiety having a strong hole mobility, which may greatly contribute to the long life-span characteristics.

For example, $L^1$ may be one selected from the linking groups of Group I.

[Group Ⅰ]

L-1 L-2 L-3 L-4 L-5 L-6

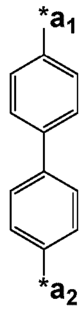

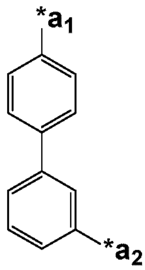

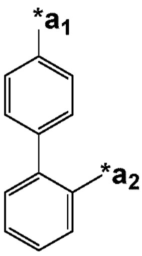

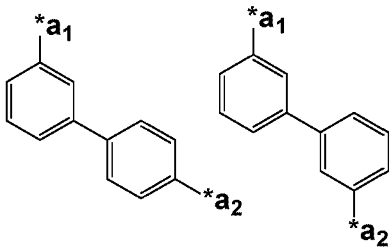

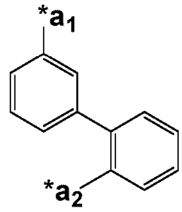

In Group I, $*a_1$ is a linking portion with triazine of Chemical Formula 1, and $*a_2$ is a linking portion with carbazole of Chemical Formula 1.

Particularly, the biphenylene may be a combination of para and meta linkages, for example the $L^1$ may be represented as L-2 or L-4.

For example, $Ar^1$ is a substituted or unsubstituted phenyl group or a substituted or unsubstituted biphenyl group.

For example, $Ar^1$ may be a phenyl group unsubstituted or substituted with deuterium (D), a phenyl group unsubstituted or substituted with a cyano group, a phenyl group unsubstituted or substituted with a C1 to C5 alkylsilyl group, a biphenyl group unsubstituted or substituted with deuterium (D), a biphenyl group unsubstituted or substituted with a cyano group, or a biphenyl group unsubstituted or substituted with a C1 to C5 alkylsilyl group, and according to an embodiment, $Ar^1$ may be a phenyl group unsubstituted or substituted with deuterium, a phenyl group unsubstituted or substituted with a cyano group, a phenyl group unsubstituted or substituted with a C1 to C5 alkylsilyl group, or an unsubstituted biphenyl group.

In the most specific embodiment, $Ar^1$ may be an unsubstituted phenyl group, but is not limited thereto.

For example, $R^1$ to $R^4$ is independently hydrogen or deuterium, and

for example, $R^1$ to $R^4$ is independently hydrogen, and
in the most specific embodiment, $R^1$ to $R^4$ may be all hydrogen, but are not limited thereto.

For example, $R^5$ to $R^8$ is independently hydrogen or deuterium,

In the most specific embodiment, $R^5$ to $R^8$ may be all hydrogen, but are not limited thereto.

For a specific example, $Ar^1$ may be an unsubstituted phenyl group and $R^5$ to $R^8$ may be all hydrogen.

For example, the compound for the organic optoelectronic device represented by Chemical Formula 1 may be one of compounds of Group 1, but is not limited thereto.

[Group 1]

A-1

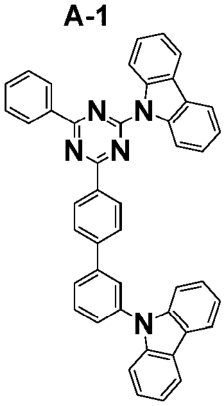

A-2

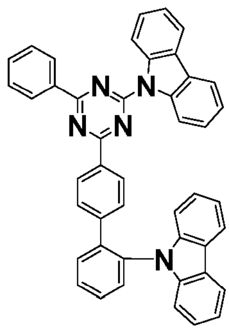

A-3

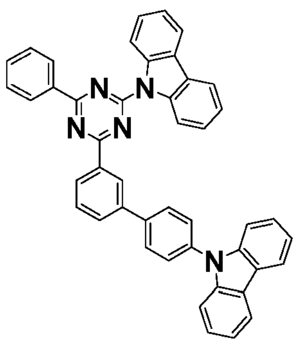

A-4

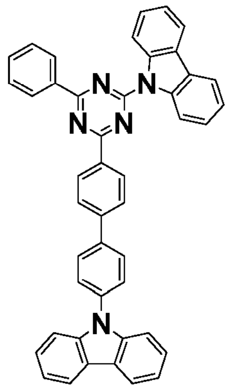

A-5

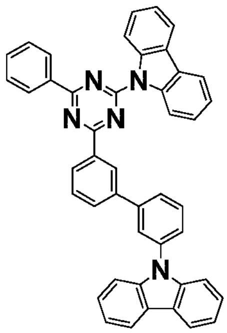

A-6

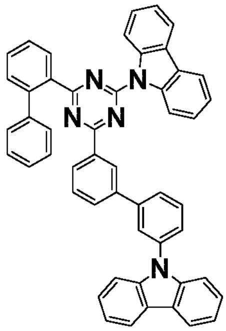

A-7

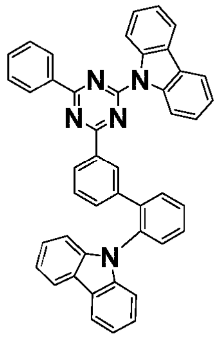

A-8

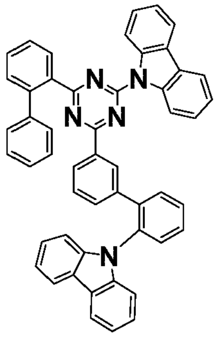

A-9

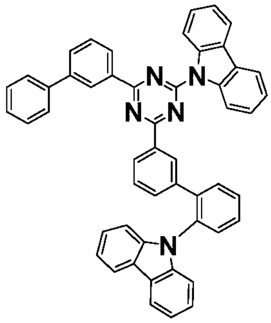

A-10

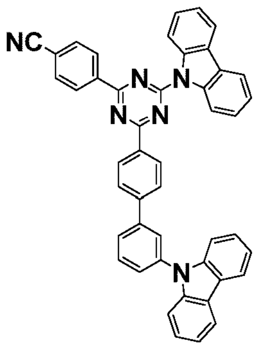

A-11

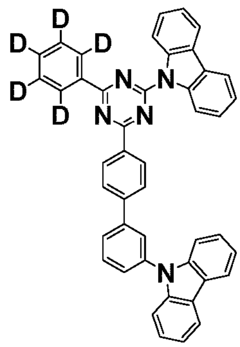

A-12

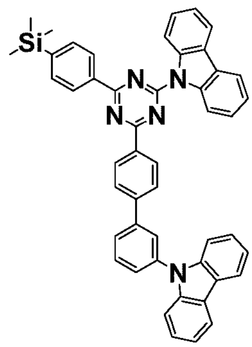

A-13

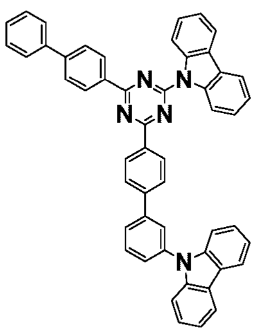

A-14

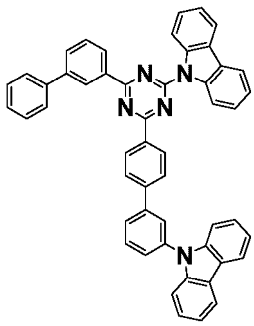

A-15

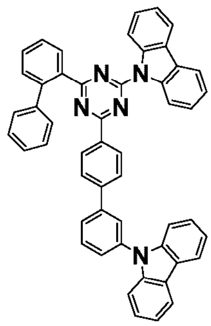

A composition for an organic optoelectronic device according to another embodiment includes a first compound for an organic optoelectronic device and a second compound for an organic optoelectronic device, wherein the first compound for the organic optoelectronic device is represented by Chemical Formula 1 and the second compound for the organic optoelectronic device is represented by Chemical Formula 2; or a combination of Chemical Formula 3 and Chemical Formula 4.

[Chemical Formula 2]

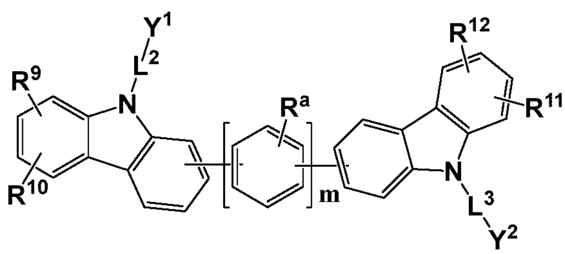

In Chemical Formula 2,

$Y^1$ and $Y^2$ are independently a substituted or unsubstituted C6 to C20 aryl group or a substituted or unsubstituted C2 to C30 heterocyclic group,
$L^2$ and $L^3$ are independently a single bond, or a substituted or unsubstituted C6 to C20 arylene group,
$R^a$ and $R^9$ to $R^{12}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, and
m is an integer of 0 to 2;

[Chemical Formula 3] [Chemical Formula 4]

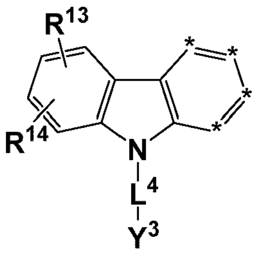

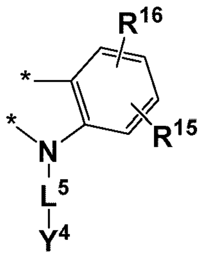

In Chemical Formula 3 and Chemical Formula 4,

$Y^3$ and $Y^4$ are independently a substituted or unsubstituted C6 to C20 aryl group or a substituted or unsubstituted C2 to C30 heterocyclic group,
adjacent two *'s of Chemical Formula 3 is linked with Chemical Formula 4,
* of Chemical Formula 3 that are not linked with Chemical Formula 4 are independently C-$L^a$-$R^b$,
$L^a$, $L^4$, and $L^5$ are independently a single bond, or a substituted or unsubstituted C6 to C20 arylene group, and
$R^b$ and $R^{13}$ to $R^{16}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

The second compound for the organic optoelectronic device may be used in a light emitting layer with the first compound for the organic optoelectronic device to improve luminous efficiency and life-span characteristics by increasing charge mobility and stability.
For example, $Y^1$ and $Y^2$ of Chemical Formula 2 may independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, a substituted or unsubstituted fluorenyl group, or a substituted or unsubstituted pyridinyl group,

$L^2$ and $L^3$ of Chemical Formula 2 may independently be a single bond, a substituted or unsubstituted phenylene group, or a substituted or unsubstituted biphenylene group,
$R^9$ to $R^{12}$ of Chemical Formula 2 may independently be hydrogen, deuterium, or a substituted or unsubstituted C6 to C12 aryl group, and
m may be 0 or 1.

For example, "substituted" of Chemical Formula 2 refers to replacement of at least one hydrogen by deuterium, a C1 to C4 alkyl group, a C6 to C18 aryl group, or a C2 to C30 heteroaryl group.
In a specific embodiment of the present invention, Chemical Formula 2 may be represented by one of Chemical Formula 2-1 to Chemical Formula 2-15.

[Chemical Formula 2-1] [Chemical Formula 2-2] [Chemical Formula 2-3]

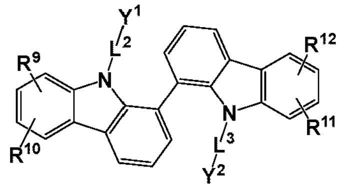
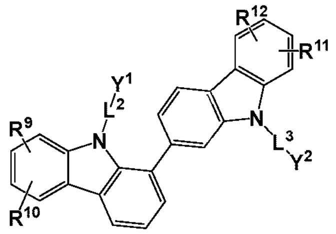
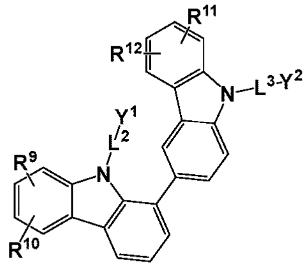

[Chemical Formula 2-4] [Chemical Formula 2-5] [Chemical Formula 2-6]

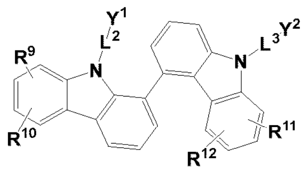
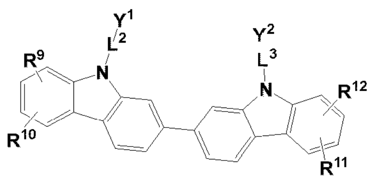
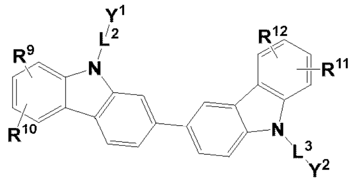

[Chemical Formula 2-7] [Chemical Formula 2-8] [Chemical Formula 2-9]

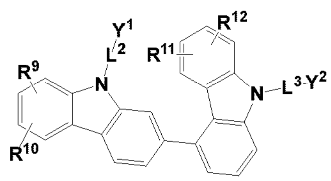
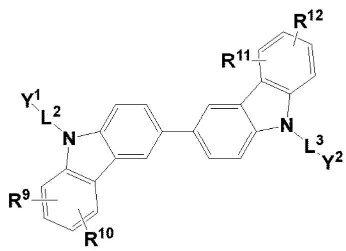
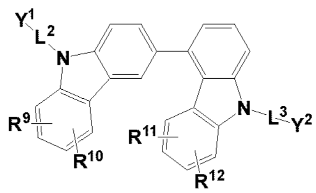

[Chemical Formula 2-10] [Chemical Formula 2-11] [Chemical Formula 2-12]

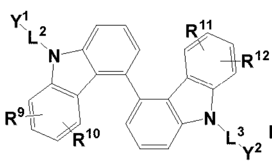
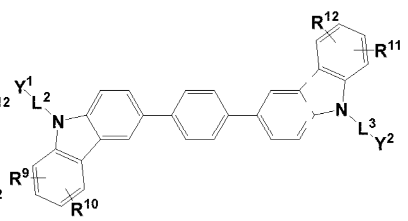
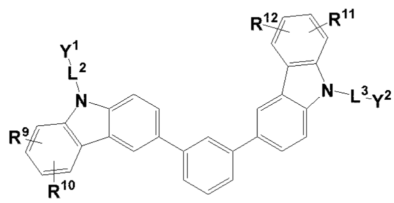

[Chemical Formula 2-13] [Chemical Formula 2-14] [Chemical Formula 2-15]

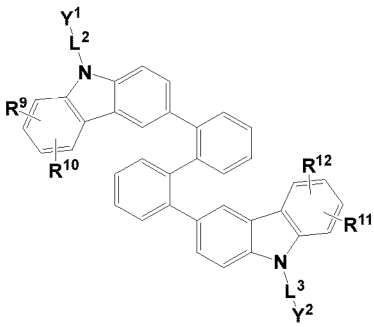

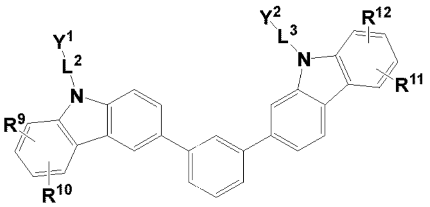

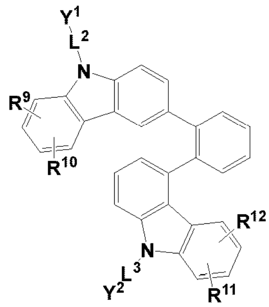

In Chemical Formula 2-1 to Chemical Formula 2-15, $R^9$ to $R^{12}$ may independently be hydrogen, or a substituted or unsubstituted C6 to C12 aryl group and $*-L^2-Y^1$ and $*-L^3-Y^2$ may independently be one of substituents of Group II.

[Group II]

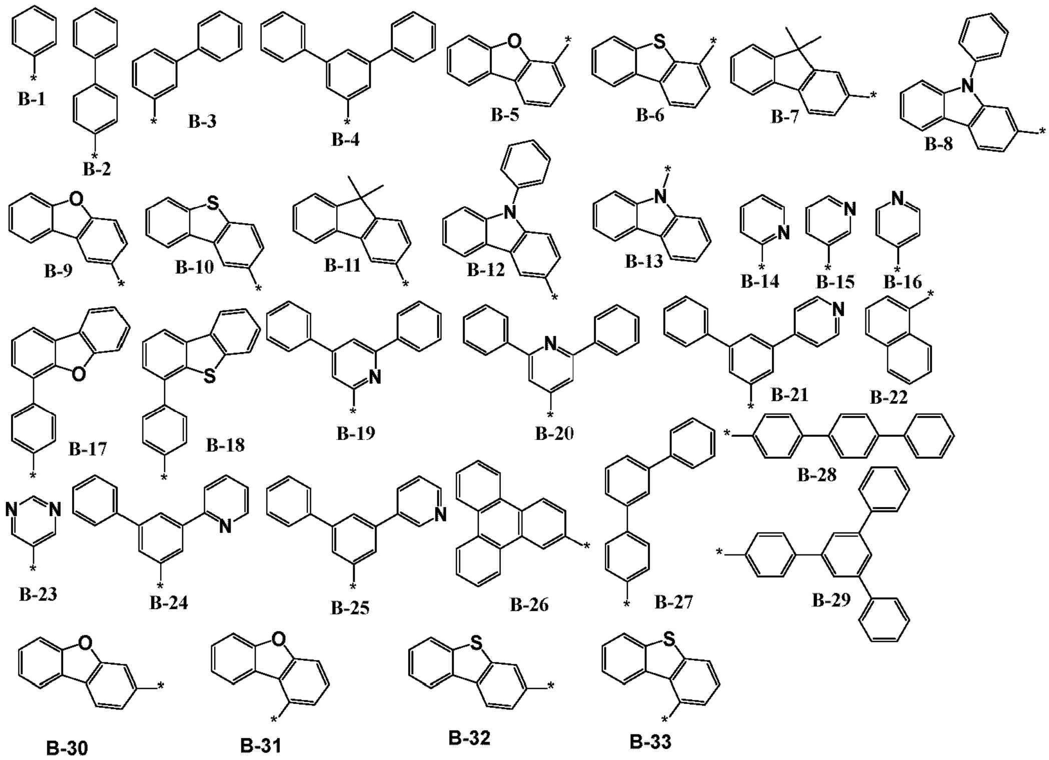

B-1 B-2 B-3 B-4 B-5 B-6 B-7 B-8 B-9 B-10 B-11 B-12 B-13 B-14 B-15 B-16 B-17 B-18 B-19 B-20 B-21 B-22 B-23 B-24 B-25 B-26 B-27 B-28 B-29 B-30 B-31 B-32 B-33

In Group II, * is a linking portion.

In an embodiment, Chemical Formula 2 may be represented by Chemical Formula 2-8.

In addition, *-$L^1$-$Y^1$ and *-$L^2$-$Y^2$ of Chemical Formula 2-8 may independently be Group II, and may be one of for example B-1, B-2, and B-3.

In the most specific embodiment, *-$L^1$-$Y^1$ and *-$L^2$-$Y^2$ may be all represented by B-2 of Group II, but is not limited thereto.

For example, the second compound for the organic optoelectronic device represented by the combination of Chemical Formula 3 and Chemical Formula 4 may be represented by one of Chemical Formula 3A, Chemical Formula 3B, Chemical Formula 3C, Chemical Formula 3D, and Chemical Formula 3E.

[Chemical Formula 3A] [Chemical Formula 3B] [Chemical Formula 3C]

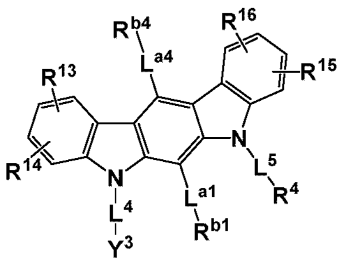
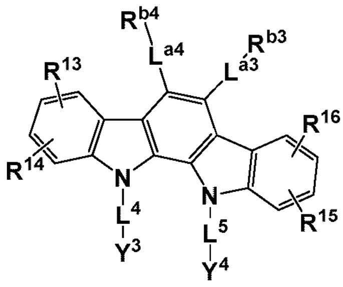
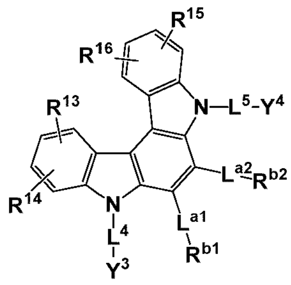

[Chemical Formula 3D] [Chemical Formula 3E]

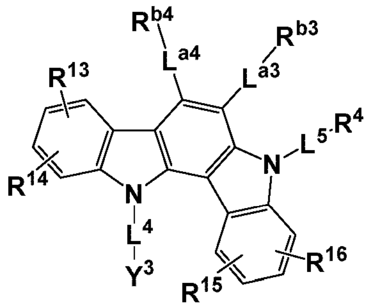
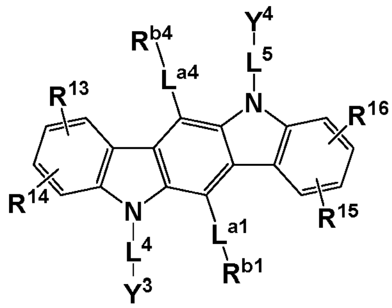

In Chemical Formula 3A to Chemical Formula 3E, $Y^3$ and $Y^4$, $L^4$ and $L^5$, and $R^{13}$ to $R^{16}$ are the same as described above,

$L^{a1}$ to $L^{a4}$ are the same as $L^4$ and $L^5$ described above, and
$R^{b1}$ to $R^{b4}$ are the same as $R^{13}$ to $R^{16}$ described above.

For example, $Y^3$ and $Y^4$ of Chemical Formulae 3 and 4 may independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, and

$R^{b1}$ to $R^{b4}$ and $R^{13}$ to $R^{16}$ may independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, $Y^3$ and $Y^4$ of Chemical Formulae 3 and 4 may independently be selected from substituents of Group III.

[Group III]

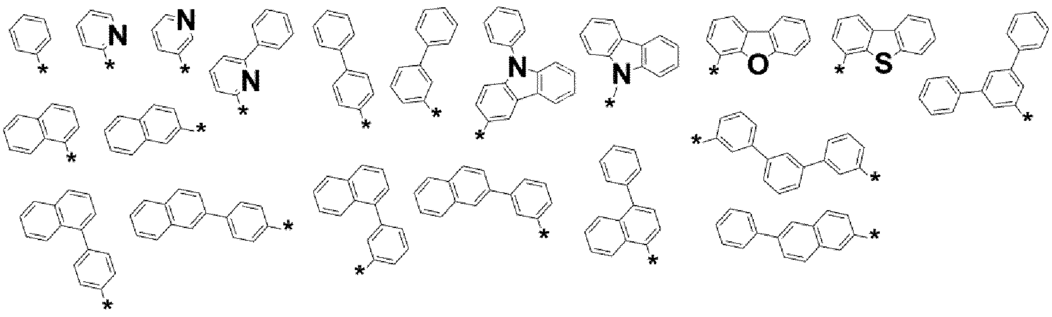

In Group III, * is a linking portion with $L^4$ and $L^5$, respectively.

In an embodiment, $R^{b1}$ to $R^{b4}$ and $R^{13}$ to $R^{16}$ may independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, $R^{b1}$ to $R^{b4}$ and $R^{13}$ to $R^{16}$ may independently be hydrogen, deuterium, a cyano group, or a substituted or unsubstituted phenyl group, and

in a specific embodiment, $R^{b1}$ to $R^{b4}$ may independently be hydrogen, and $R^{13}$ to $R^{16}$ may independently be hydrogen or a phenyl group.

In a specific embodiment of the present invention, the second compound for the organic optoelectronic device may be represented by Chemical Formula 2-8 or Chemical Formula 3C.

Herein, $Y^1$ to $Y^4$ of Chemical Formula 2-8 and Chemical Formula 3C may independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, $L^2$ to $L^5$, $L^{a1}$ and $L^{a2}$ may independently be a single bond, or a substituted or unsubstituted C6 to C20 arylene group, and $R^{b1}$, $R^{b2}$ and $R^9$ to $R^{16}$ may independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In the most specific embodiment, $*-L^2-Y^1$, $*-L^3-Y^2$, $*-L^4-Y^3$, and $*-L^5-Y^4$ of Chemical Formula 2-8 and Chemical Formula 3C may be all represented by B-2 of Group III, but are not limited thereto.

For example, the second compound for the organic optoelectronic device may be one selected from the compounds of Group 2, but is not limited thereto.

[Group 2]

[B-1] [B-2] [B-3] [B-4] [B-5]

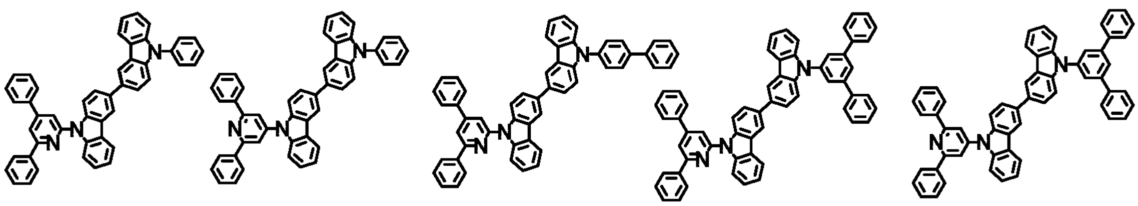

[B-6] [B-7] [B-8] [B-9] [B-10]

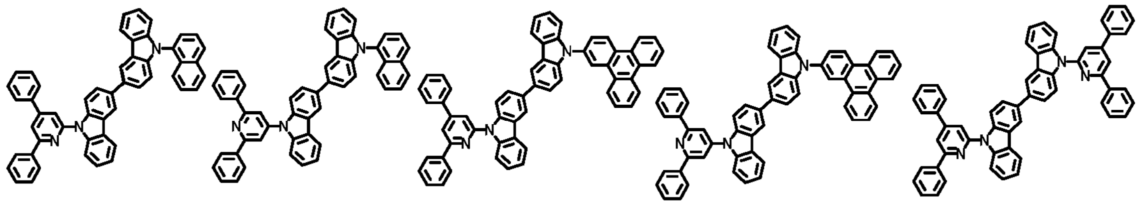

[B-11] [B-12] [B-13] [B-14] [B-15]

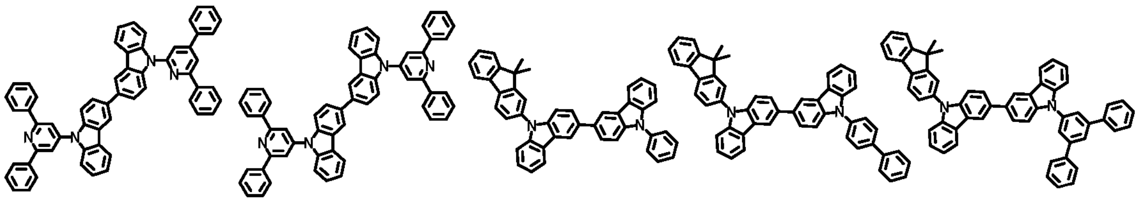

[B-16] [B-17] [B-18] [B-19] [B-20]

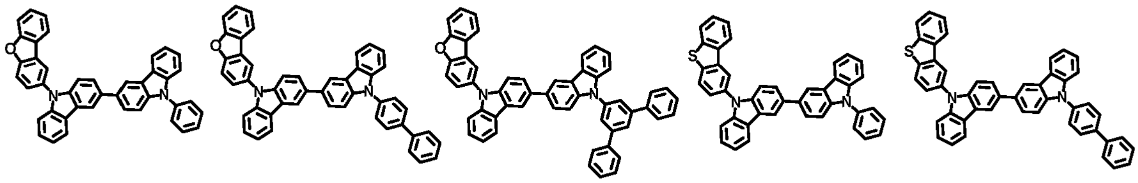

[B-21] [B-22] [B-23] [B-24] [B-25]

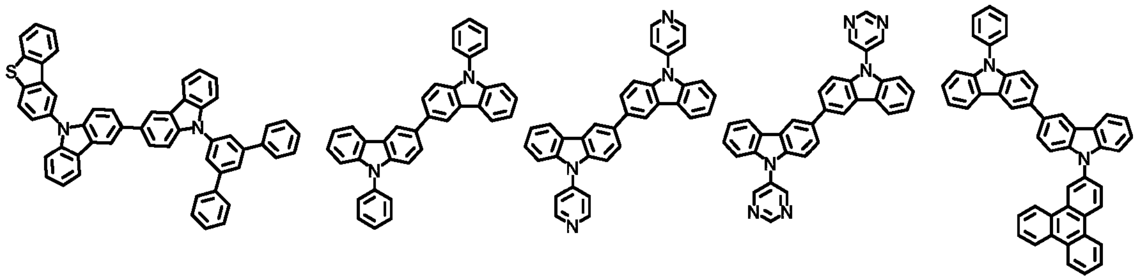

[B-26] [B-27] [B-28] [B-29] [B-30]

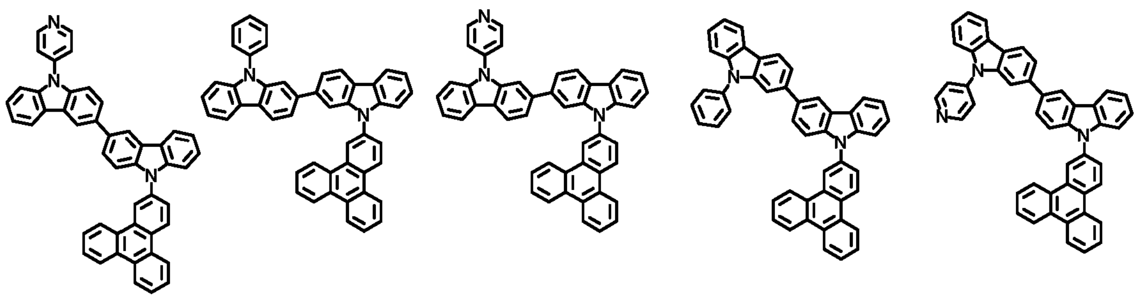

[B-31] [B-32] [B-33] [B-34] [B-35]

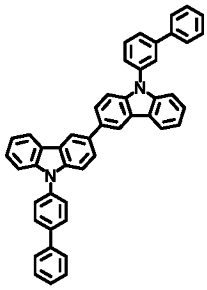
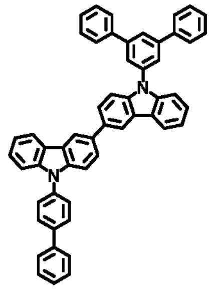
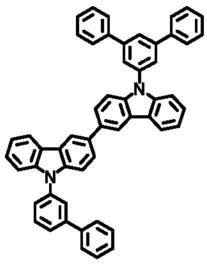
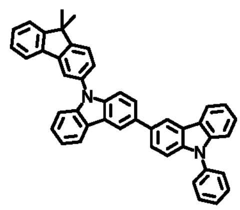
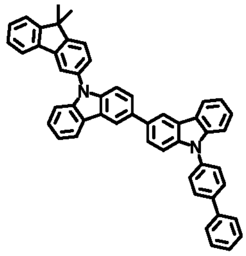

[B-36] [B-37] [B-38] [B-39] [B-40]

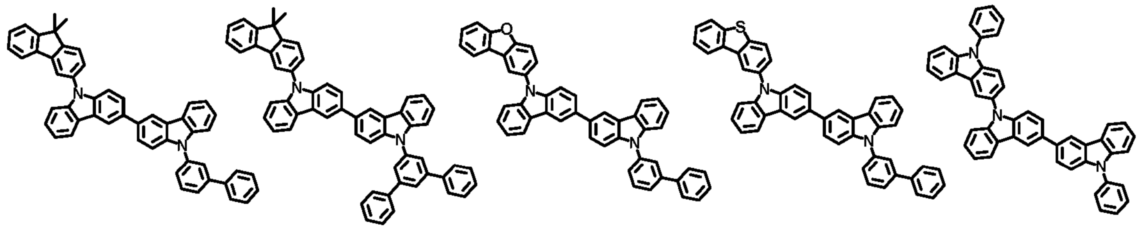

[B-41] [B-42] [B-43] [B-44] [B-45]

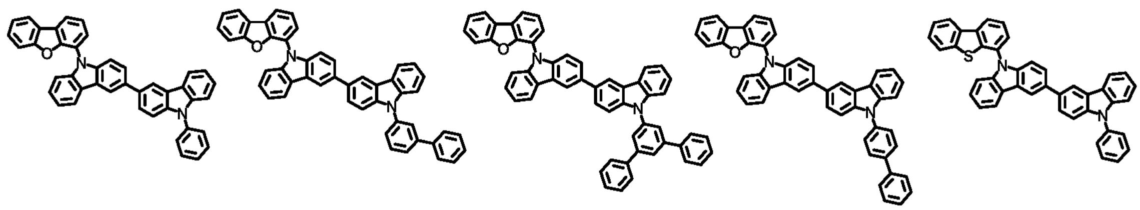

[B-46] [B-47] [B-48] [B-49] [B-50]

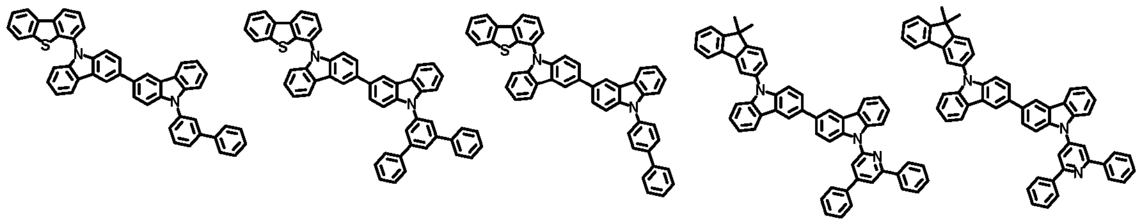

[B-51] [B-52] [B-53] [B-54] [B-55]

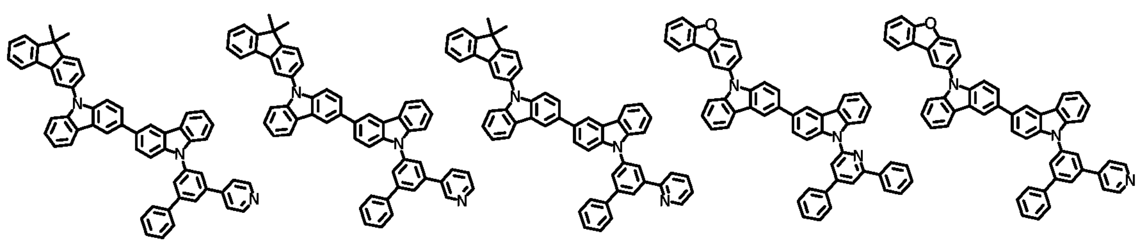

[B-56] [B-57] [B-58] [B-59] [B-60]

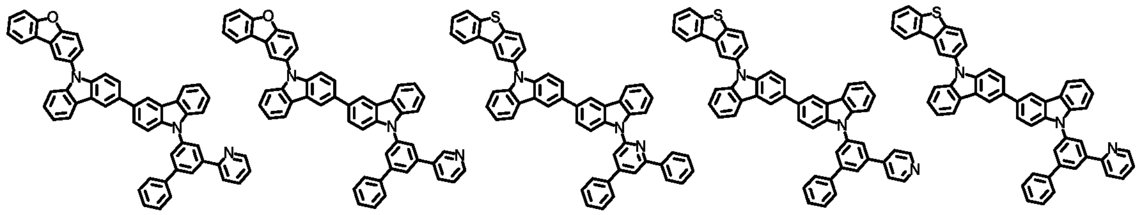

[B-61] [B-62] [B-63] [B-64] [B-65]

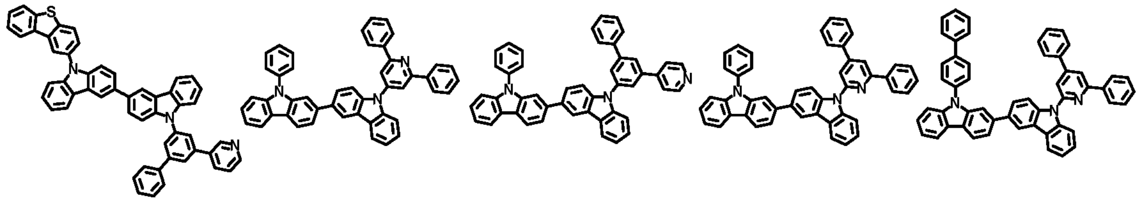

[B-66] [B-67] [B-68] [B-69] [B-70]

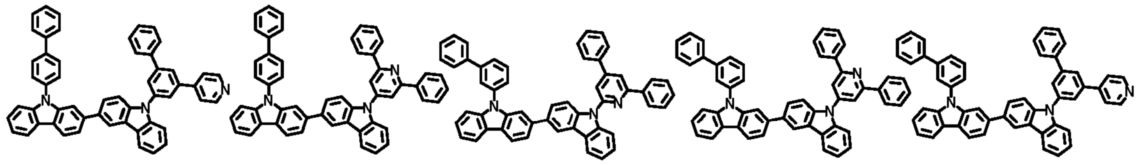

[B-71] [B-72] [B-73] [B-74] [B-75]

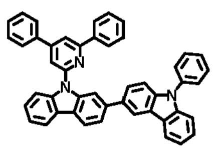
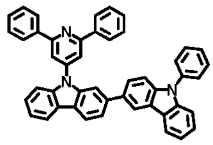
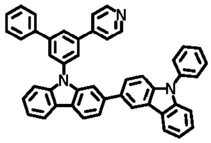
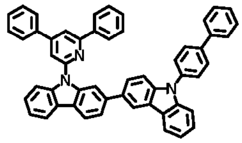
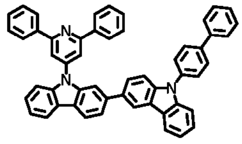

[B-76] [B-77] [B-78] [B-79] [B-80]

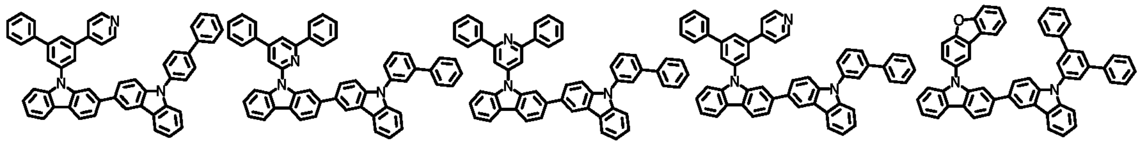

[B-81] [B-82] [B-83] [B-84] [B-85]

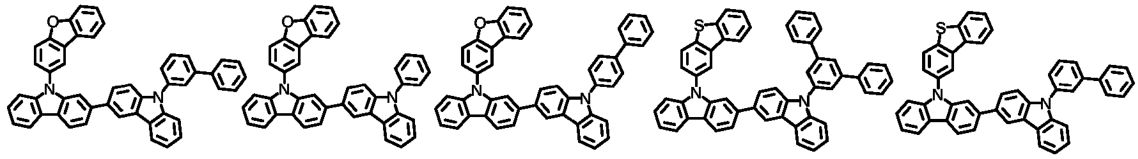

[B-86] [B-87] [B-88] [B-89] [B-90]

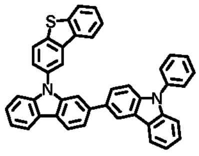 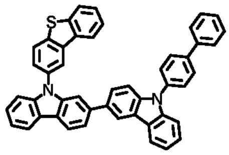 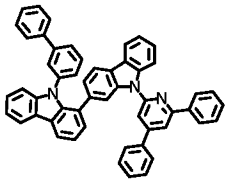 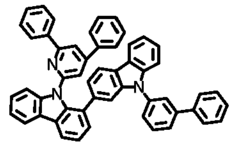 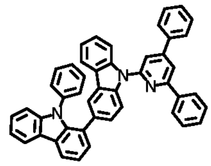

[B-91] [B-92] [B-93] [B-94] [B-95]

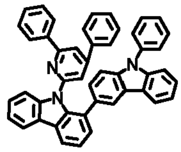 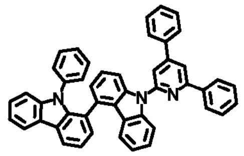 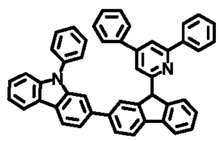 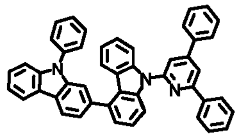 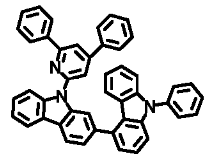

[B-96] [B-97] [B-98] [B-99] [B-100]

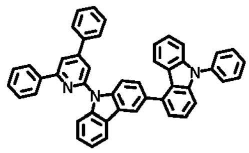 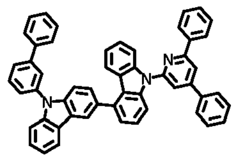 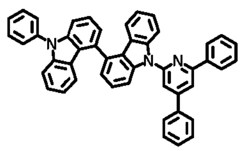 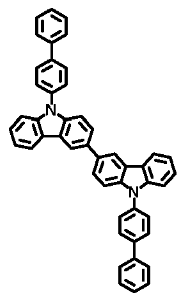 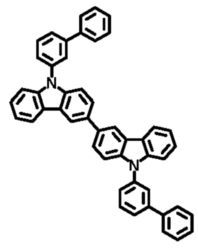

[B-101] [B-102] [B-103] [B-104] [B-105]

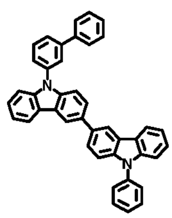 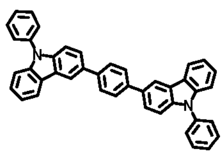 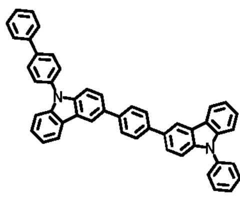 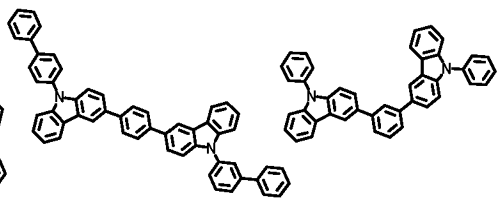

[B-106] [B-107] [B-108] [B-109] [B-110]

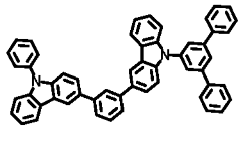 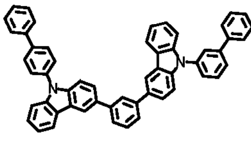 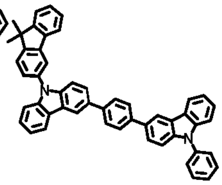 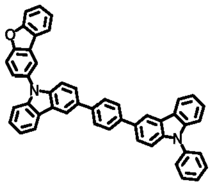 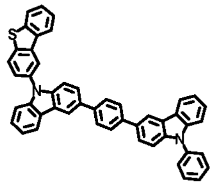

[B-111] [B-112] [B-113] [B-114] [B-115]

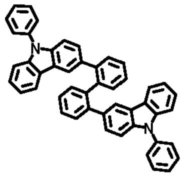
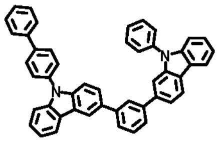
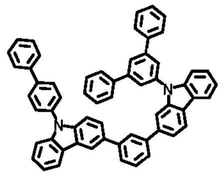
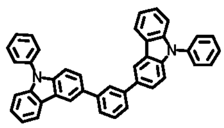
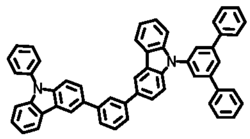

[B-116] [B-117] [B-118] [B-119] [B-120]

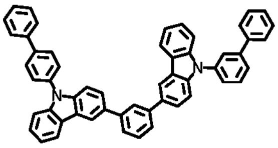
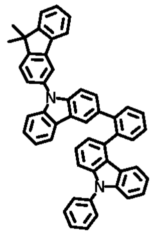
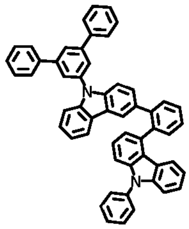
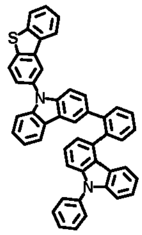
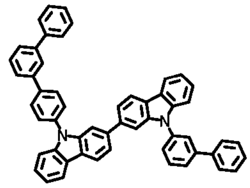

[B-121] [B-122] [B-123] [B-124] [B-125]

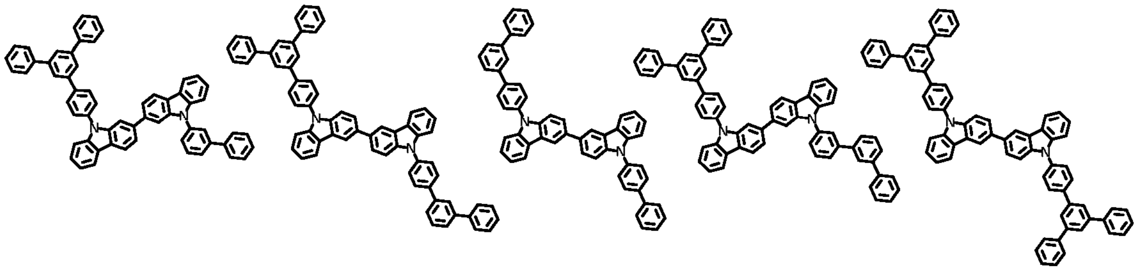

[B-126] [B-127] [B-128] [B-129] [B-130]

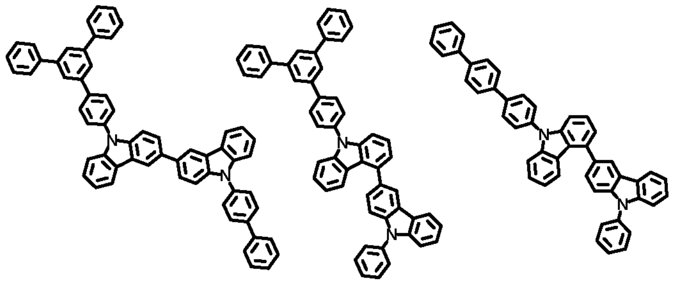
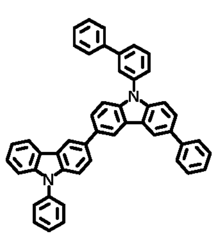
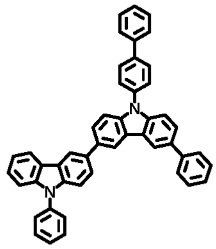

[B-131] [B-132] [B-133] [B-134] [B-135]

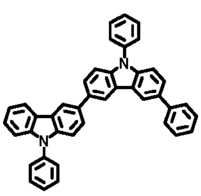
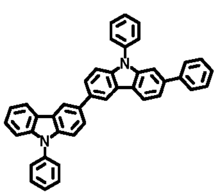
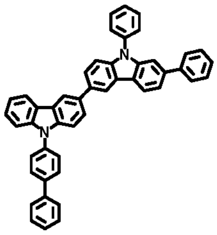
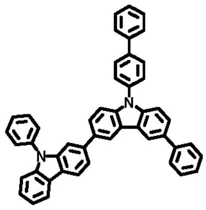
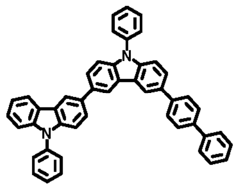

[B-136] [B-137] [B-138]

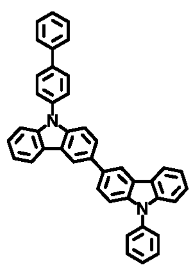 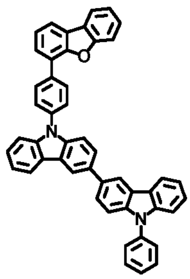 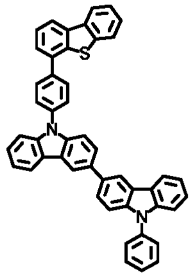

[C-1] [C-2] [C-3] [C-4]

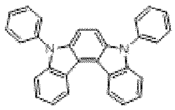 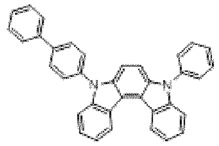 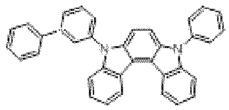 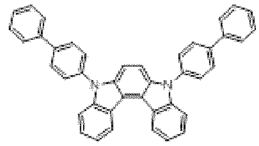

[C-5] [C-6] [C-7] [C-8]

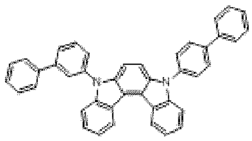 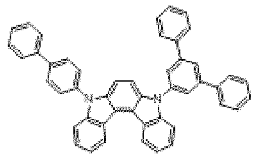 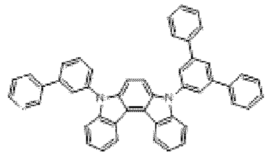 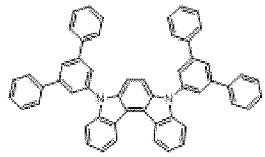

[C-9] [C-10] [C-11] [C-12]

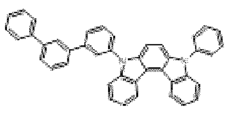 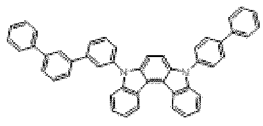 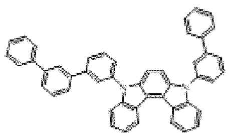 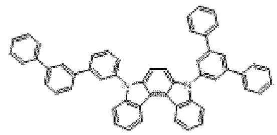

[C-13] [C-14] [C-15] [C-16]

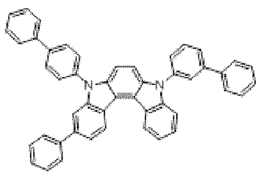 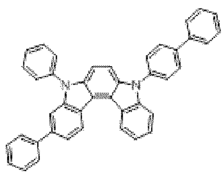 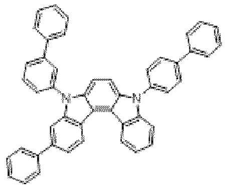 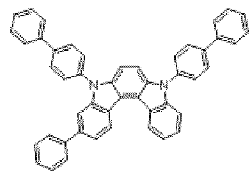

[C-17] [C-18] [C-19] [C-20]

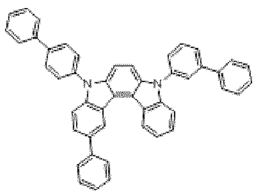 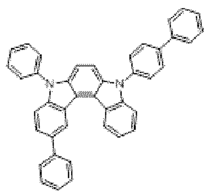 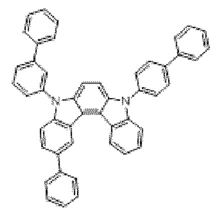 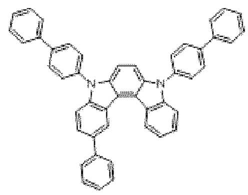

[C-21] [C-22] [C-23] [C-24]

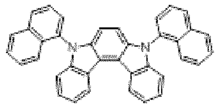 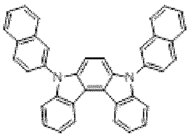 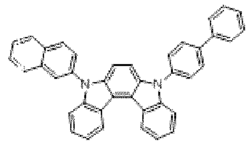 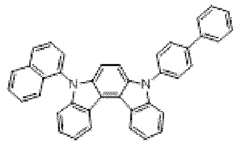

[C-25] [C-26] [C-27] [C-28]

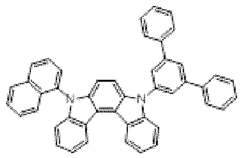 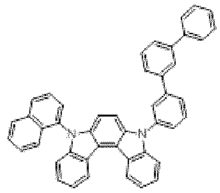 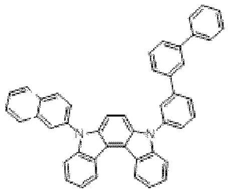 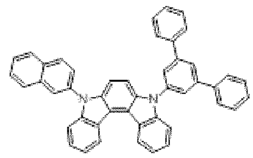

[C-29] [C-30] [C-31] [C-32]

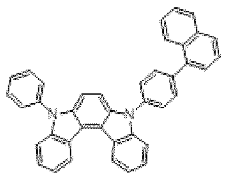 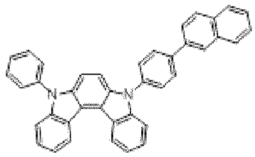 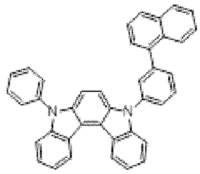 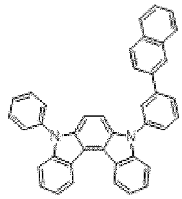

[C-33] [C-34] [C-35] [C-36]

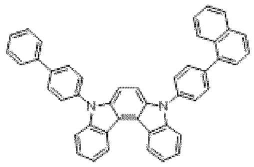 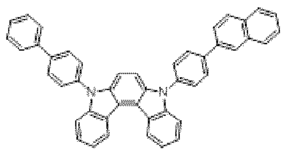 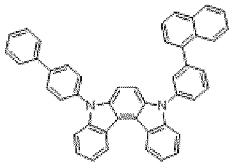 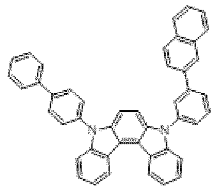

[C-37] [C-38] [C-39] [C-40]

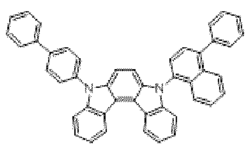 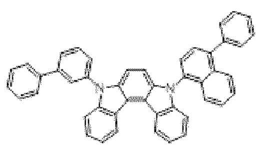 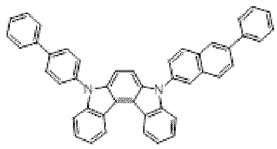 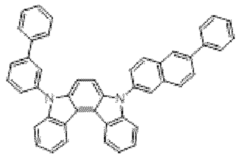

[C-41] [C-42] [C-43] [C-44]

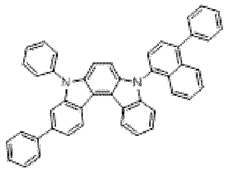 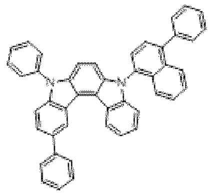 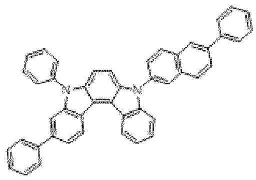 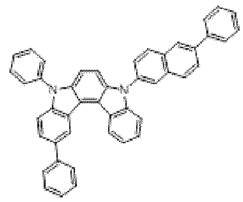

[C-45] [C-46] [C-47] [C-48]

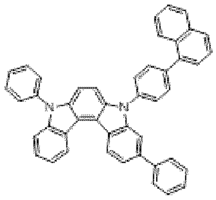 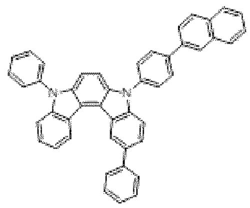 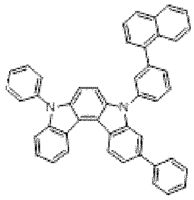 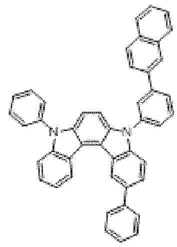

[C-49] [C-50] [C-51] [C-52]

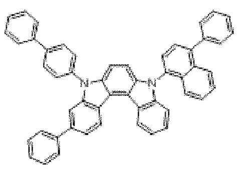 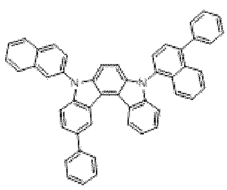 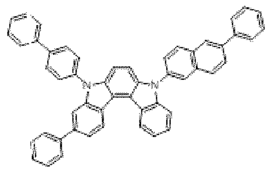 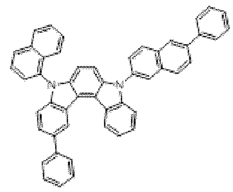

[C-53] [C-54] [C-55] [C-56]

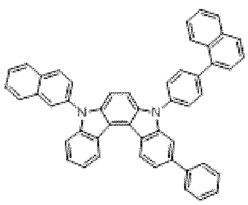 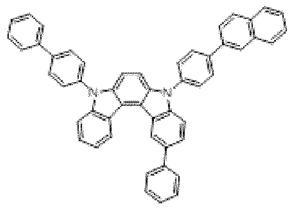 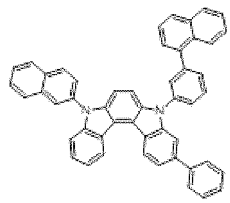 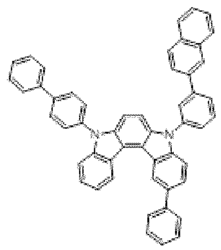

The first compound for the organic optoelectronic device and the second compound for the organic optoelectronic device may be for example included in a weight ratio of 1:99 to 99:1. Within the range, a desirable weight ratio may be adjusted using an electron transport capability of the first compound for the organic optoelectronic device and a hole transport capability of the second compound for the organic optoelectronic device to realize bipolar characteristics and thus to improve efficiency and life-span. Within the range, it may be, for example, a weight ratio of about 10:90 to 90:10, about 20:80 to 80:20, for example a weight ratio of about 20:80 to about 70:30, about 20:80 to about 60:40, and about 20:80 to about 50:50. For example, the weight ratio may be a weight ratio of 20:80 to 40:60 and for a specific example, the weight ratio may be a weight ratio of 30:70, 40:60, or 50:50. More specifically, it may be a weight ratio of 30:70 or 50:50.

At least one compound may be included in addition to the first compound for the organic optoelectronic device and second compound for the organic optoelectronic device.

The compound for the organic optoelectronic device or the composition for the organic optoelectronic device may be a composition that further includes a dopant.

The dopant may be for example a phosphorescent dopant, may be for example red, green, or blue phosphorescent dopant, may be for example red or green phosphorescent dopant.

The dopant is mixed in a small amount in the compound or composition for the organic optoelectronic device to cause light emission, and may be generally a material such as a metal complex that emits light by multiple excitation into a triplet or more. The dopant may be, for example an inorganic, organic, or organic/inorganic compound, and one or more kinds thereof may be used.

The dopant may be for example a phosphorescent dopant and examples of the phosphorescent dopant may be an organometallic compound including Ir, Pt, Os, Ti, Zr, Hf, Eu, Tb, Tm, Fe, Co, Ni, Ru, Rh, Pd, or a combination thereof. The phosphorescent dopant may be for example a compound represented by Chemical Formula Z, but is not limited thereto.

[Chemical Formula Z] $L^5MX^a$

In Chemical Formula Z, M is a metal, and $L^5$ and $X^a$ are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof and L

and X may be for example a bidendate ligand.

The compound for the organic optoelectronic device or the composition for the organic optoelectronic device may be formed by a dry film deposition such as chemical vapor deposition (CVD).

Hereinafter, an organic optoelectronic device including the compound for the organic optoelectronic device or the composition for the organic optoelectronic device is described.

The organic optoelectronic device may be any element to convert electrical energy into photoenergy and vice versa without particular limitation, and may be, for example an organic photoelectric device, an organic light emitting diode, an organic solar cell, and an organic photo conductor drum.

Herein, an organic light emitting diode as one example of an organic optoelectronic device is described referring to drawings.

FIGS. 1 and 2 are cross-sectional views of an organic light emitting diode according to embodiments.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection and may be for example made of a metal, a metal oxide and/or a conductive polymer. The anode 120 may be, for example a metal such as nickel, platinum, vanadium, chromium, copper, zinc, gold, and the like, or an alloy thereof; a metal oxide such as zinc oxide, indium oxide, indium tin oxide (ITO), indium zinc oxide (IZO), and the like; a combination of a metal and an oxide such as ZnO and Al or $SnO_2$ and Sb; a conductive polymer such as poly(3-methylthiophene), poly(3,4-(ethylene-1,2-dioxy) thiophene) (PEDOT), polypyrrole, and polyaniline, but is not limited thereto.

The cathode 110 may be made of a conductor having a small work function to help electron injection, and may be for example made of a metal, a metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, $LiO_2$/Al, LiF/Ca, LiF/Al, and $BaF_2$/Ca, but is not limited thereto.

The organic layer 105 may include the compound for the organic optoelectronic device or the composition for the organic optoelectronic device.

The organic layer 105 may include a light emitting layer 130 and the light emitting layer 130 may include the compound for the organic optoelectronic device or the composition for the organic optoelectronic device.

The composition for the organic optoelectronic device further including a dopant may be for example a green light emitting composition.

The light emitting layer 130 may include the first compound for the organic optoelectronic device and the second compound for the organic optoelectronic device described above as an example of a phosphorescent host.

The organic layer may further include an auxiliary layer in addition to the light emitting layer.

The auxiliary layer may be for example a hole auxiliary layer 140.

Referring to FIG. 2, an organic light emitting diode 200 further include a hole auxiliary layer 140 in addition to the light emitting layer 130. The hole auxiliary layer 140 may further increase hole injection and/or hole mobility and block electrons between the anode 120 and the light emitting layer 130.

The hole auxiliary layer 140 may include, for example, at least one of the compounds of Group A.

In more detail, the hole auxiliary layer 140 may include a hole transport layer between the anode 120 and the light emitting layer 130 and a hole transport auxiliary layer between the light emitting layer 130 and the hole transport layer, and at least one of the compounds of Group D may be included in the hole transport auxiliary layer.

[Group D]

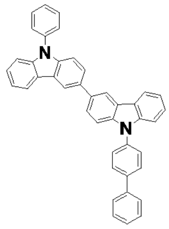
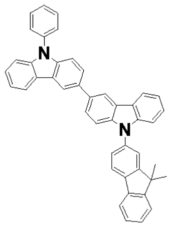
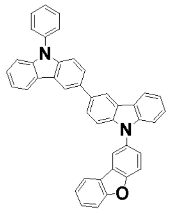
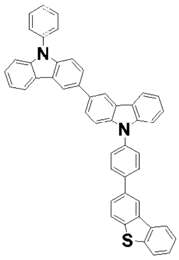
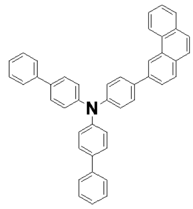
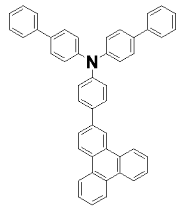

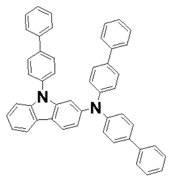
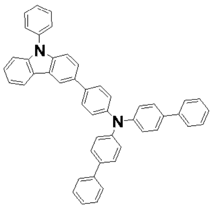
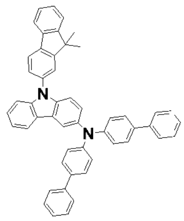
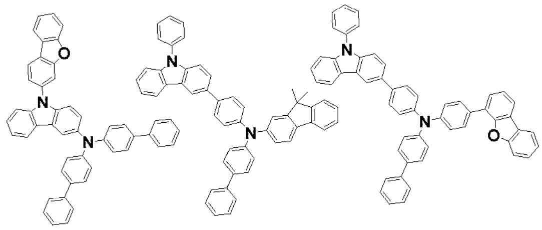

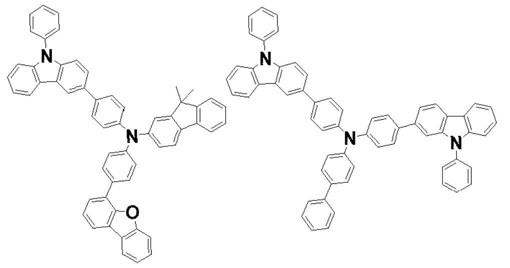
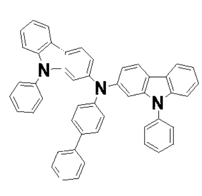
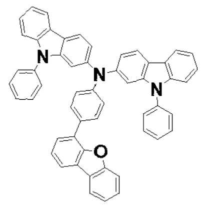
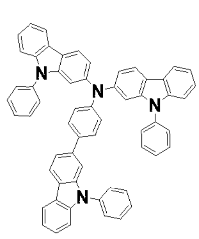
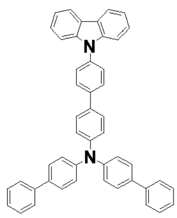
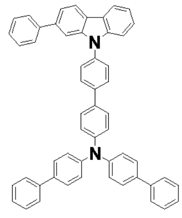
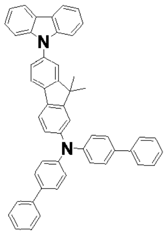
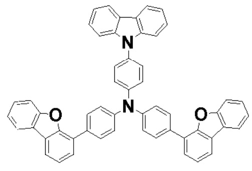
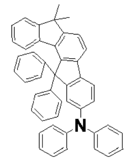
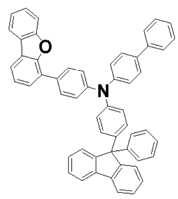
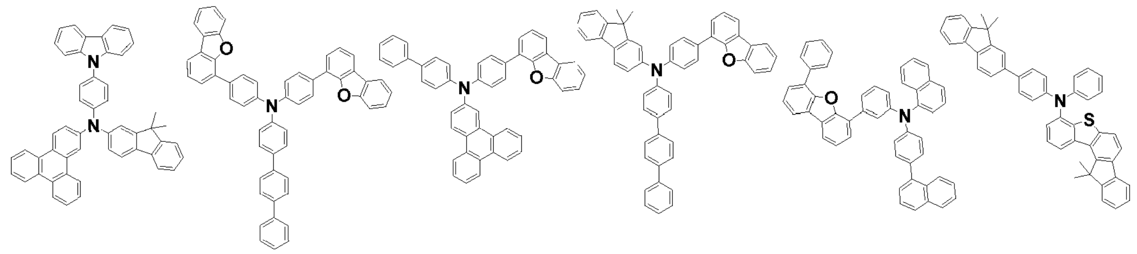
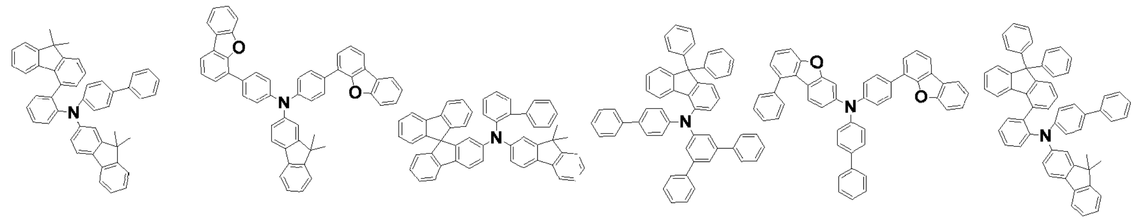
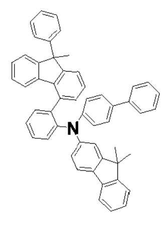
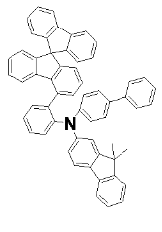
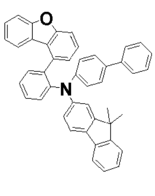
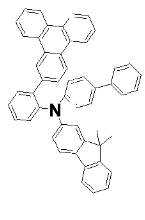
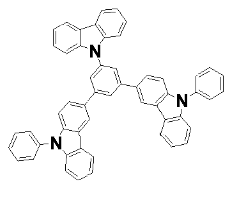
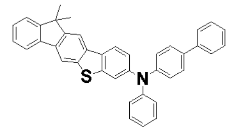
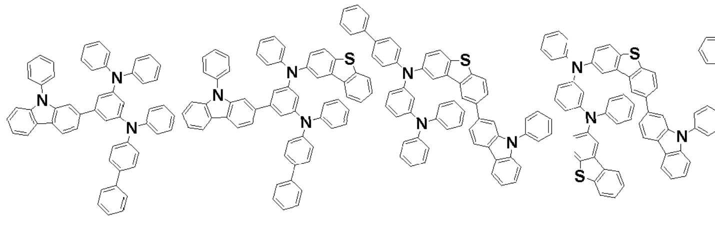
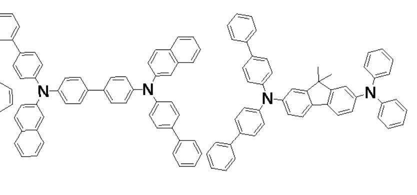

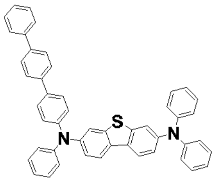 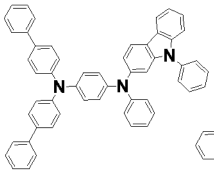 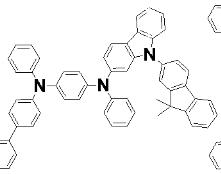 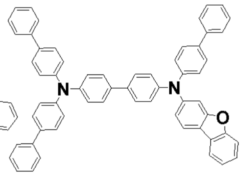 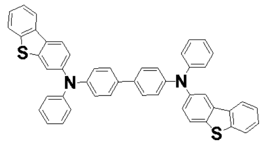

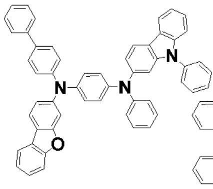 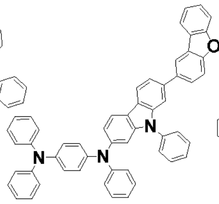 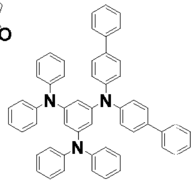 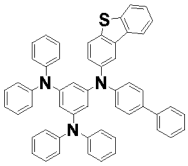 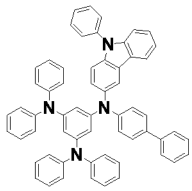 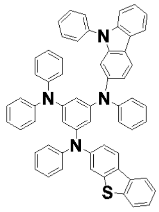

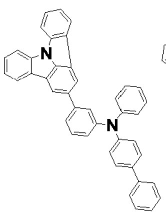 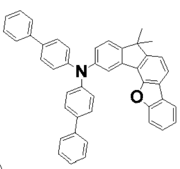 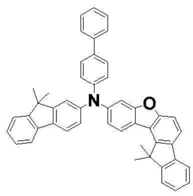 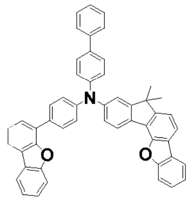 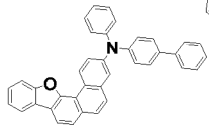 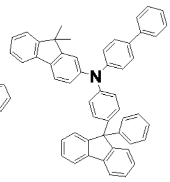

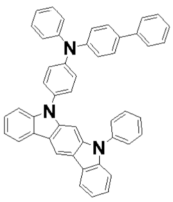 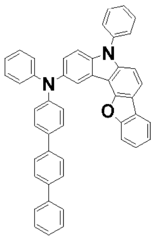 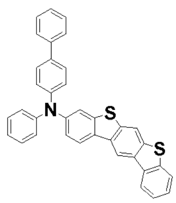 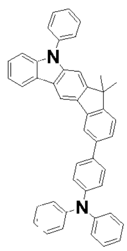 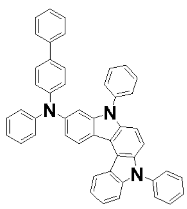 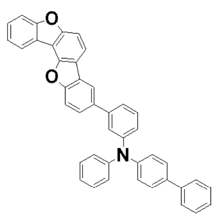

In addition to the compounds described above, the hole transport auxiliary layer may also include known compounds of US5061569A, JP1993-009471A, WO1995-009147A1, JP1995-126615A, JP1998-095973A, and the like, and compounds having similar structures.

In an embodiment, in FIG. 1 or 2, an organic light emitting diode may further include an electron transport layer, an electron injection layer, or a hole injection layer as the organic layer 105.

The organic light emitting diodes 100 and 200 may be manufactured by forming an anode or a cathode on a substrate, forming an organic layer using a dry film formation method such as a vacuum deposition method (evaporation), sputtering, plasma plating, and ion plating, and forming a cathode or an anode thereon.

The organic light emitting diode may be applied to an organic light emitting display device.

Hereinafter, the embodiments are illustrated in more detail with reference to examples. However, these examples are exemplary, and the present scope is not limited thereto.

## Mode for Invention

Hereinafter, starting materials and reactants used in Examples and Synthesis Examples were purchased from Sigma-Aldrich Co. Ltd., TCI Inc., Tokyo Chemical Industry, or P&H Tech as far as there in no particular comment or were synthesized by known methods.

### (Preparation of Compound for Organic Optoelectronic Device)

The compound as one specific examples of the present invention was synthesized through the following steps.

### (Preparation of First Compound for Organic Optoelectronic Device)

**Synthesis Example 1: Synthesis of Intermediate A**

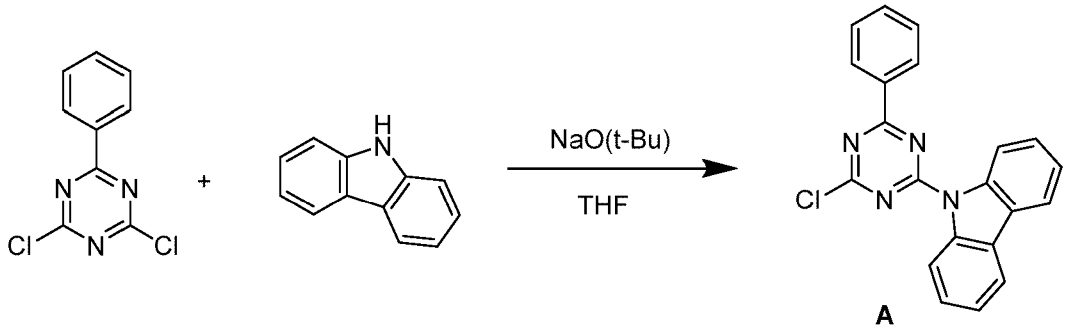


A

58.81 g (260.15 mmol) of 2-phenyl-4,6-dichlorotriazine and 30 g (179.42 mmol) of carbazole were suspended in 500 ml of THF, and 18.11 g of NaO(t-Bu) was slowly added thereto and then, stirred at room temperature for 12 hours. When a reaction was complete, a solid generated therein was filtered, washed with distilled water and acetone, and dried to obtain 40 g (a yield of 62.5 %) of Intermediate A as a target compound.
(LC / MS: Theoretical value: 356.81, Measured value: 357.30)

**Synthesis Example 2: Synthesis of Intermediate B**

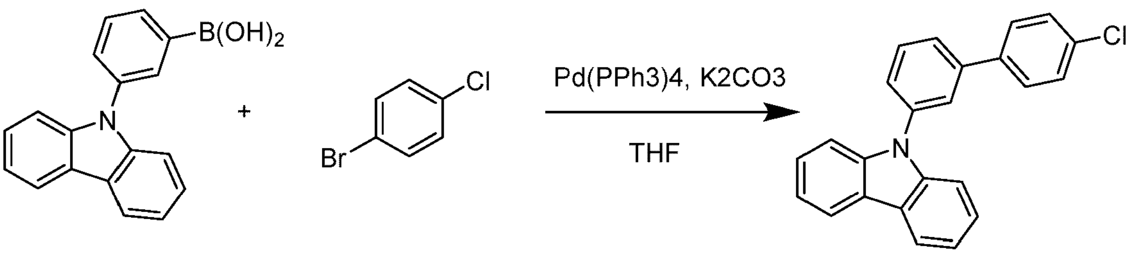


B

30.29 g (105 mmol) of 3-(9H-carbazole-9-yl)phenyl boronic acid, 20 g (105 mmol) of 1-bromo-4-chlorobenzene, 0.03 eq of $Pd(PPh_3)_4$, and 2 eq of $K_2CO_3$ were suspended in THF and distilled water and then, stirred for 12 hours. When a reaction was complete, an organic layer was extracted therefrom and then, concentrated and silica-gel columned to obtain 35 g (a yield : 94%) of Intermediate B as a target compound.

**Synthesis Example 3: Synthesis of Intermediate C**

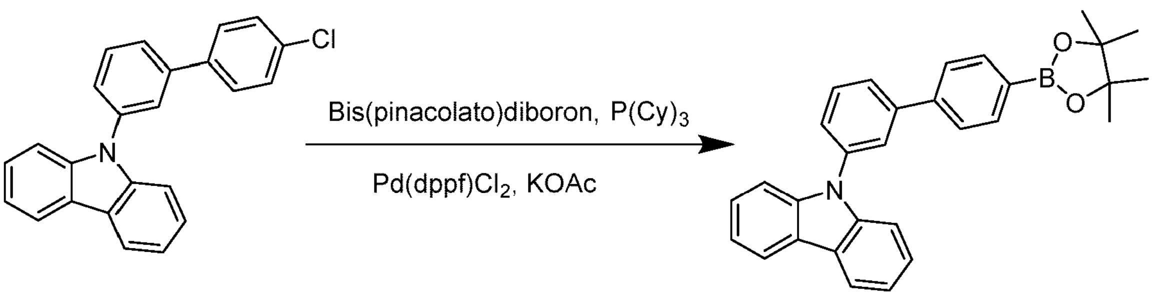


C

35 g (100 mmol) of Intermediate B synthesized according to Synthesis Example 2, 4.85 g (10 mmol) of Pd(dppf)

$Cl_2$, 29.12 g (300 mmol) of KOAc, 30.14 g (120 mmol) of bis(pinacolato)diboron, and 6.66 g (20 mmol) of $P(Cy)_3$ were suspended in 500 ml of DMF and then, refluxed and stirred for 12 hours. When a reaction was completed, distilled water was added to the reaction solution and then, extracted/concentrated with methylene chloride and silica gel-columned to obtain 30.8 g (a yield of 70 %) of intermediate C as a target compound.

## Synthesis Example 4: Synthesis of Compound A-1

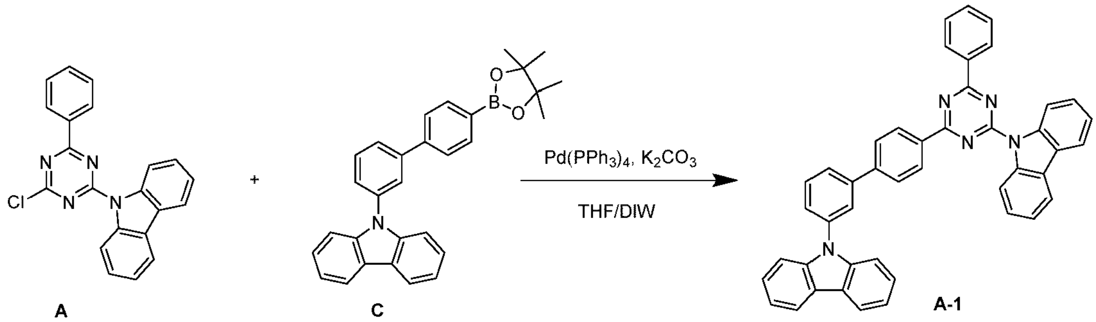


A C A-1

5 g (14.01 mmol) of Intermediate A, 6.86 g (15.41 mmol) of Intermediate C, 0.49 g (0.42 mmol) of $Pd(PPh_3)_4$, and 3.87 g (28.03 mmol) of $K_2CO_3$ were suspended in 100 ml of THF and 50 ml of distilled water and then, refluxed and stirred for 12 hours. When a reaction was complete, the reaction solution was cooled down to room temperature, and a solid generated therein was filtered and washed with distilled water and acetone. Subsequently, the solid was heated with 200 ml of toluene and dissolved therein and then, silica gel-filtered, a filtrate therefrom was cooled down to room temperature, and a solid generated therein was filtered, washed with acetone, and dried to obtain 6.7 g of Compound A-1 (a yield of 74%).
(LC/MS : Theoretical value: 639.75, Measured value: 640.45)

## Synthesis Example 5: Synthesis of Intermediate D

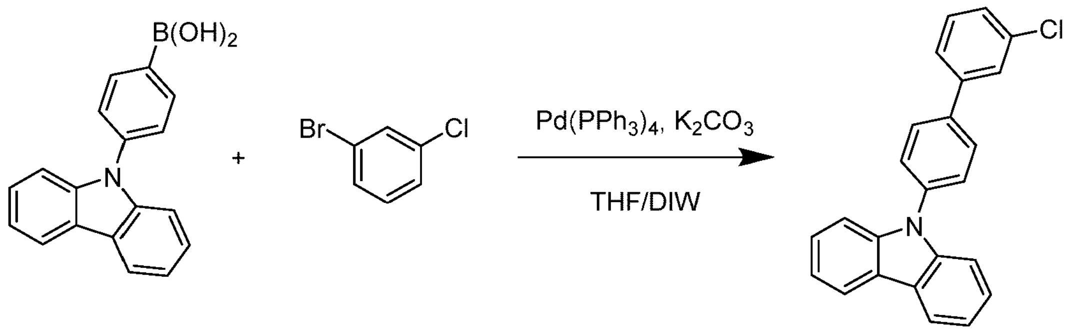


D

20 g (69.66 mmol) of 4-(9H-carbazole-9-yl)phenyl boronic acid, 13.34 g (69.66 mmol) of 1-bromo-3-chlorobenzene, 2.42 g (2.09 mmol) of $Pd(PPh_3)_4$, and 19.25 g (139.31 mmol) of $K_2CO_3$ were suspended in 300 ml of THF and 150 ml of distilled water and then, synthesized according to the same method as Synthesis Example 2 to obtain 19.82 g (a yield of 81%) of Intermediate D.

## Synthesis Example 6: Synthesis of Intermediate E

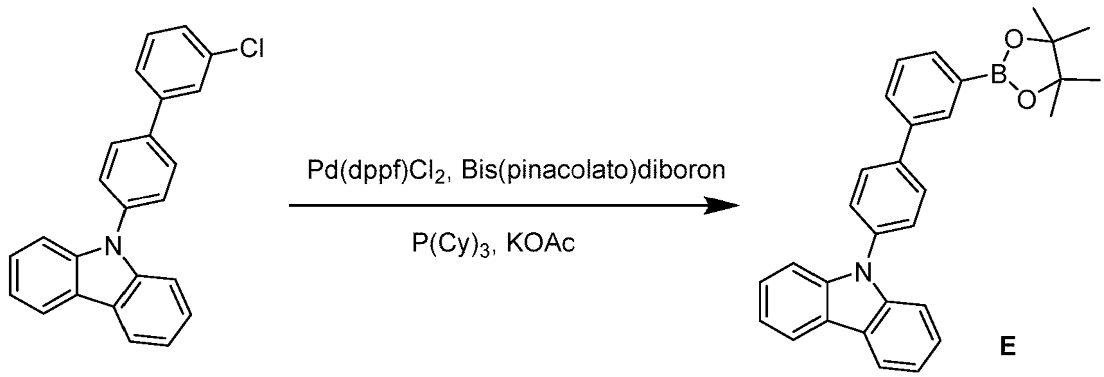


17 g (a yield of 68%) of Intermediate E was synthesized according to the same method d as Synthesis Example 3 except that 19.82 g (56.01 mmol) of Intermediate D synthesized according to Synthesis Example 5 was used.

**Synthesis Example 7: Synthesis of A-3**

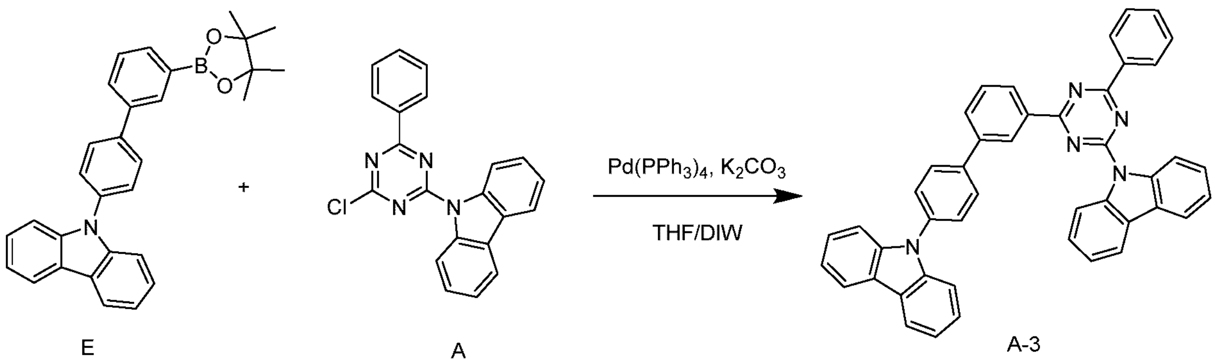


E A A-3

6 g (a yield of 74%) of Compound A-3 was synthesized according to the same method d as Synthesis Example 4 except that 4.55 g (12.7 mmol) of Intermediate A synthesized according to Synthesis Example 1 and 5.79 g (13.01 mmol) of Intermediate E synthesized according to Synthesis Example 6 were used.
(LC/MS : Theoretical value: 639.75, Measured value: 640.44)

**Synthesis Example 8: Synthesis of Comparative Compound R1**

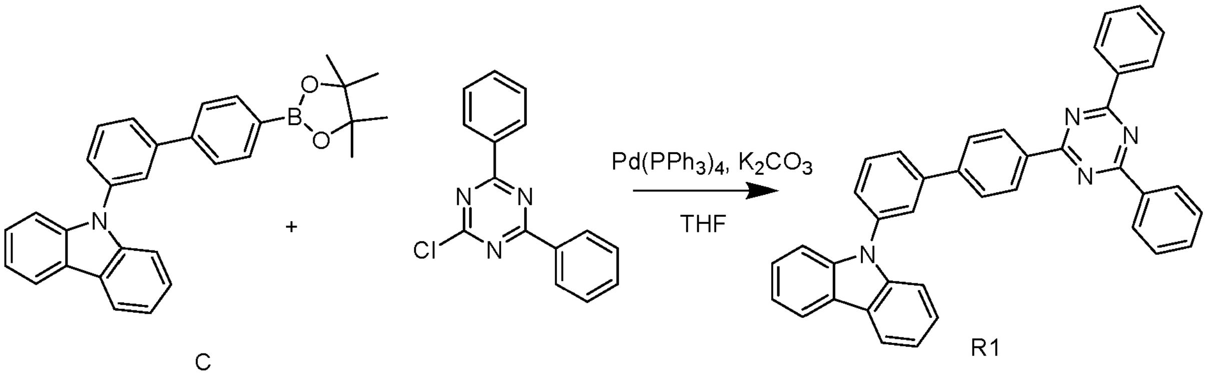


C R1

5.0 g (11.23 mmol) of Intermediate C synthesized according to Synthesis Example 3, 3 g (11.23 mmol) of 2-chloro-4,6-diphenyltriazine, 0.03 eq of Pd($PPh_3$)$_4$, and 2 eq of $K_2CO_3$ were suspended in THF and distilled water and then, refluxed and stirred for 12 hours. When a reaction was complete, a solid generated therein was filtered and washed with

distilled water and acetone. The solid was recrystallized in monochlorobenzene to obtain 4 g (a yield of 65 %) of Comparative Compound R1.
(LC/MS : Theoretical value: 550.65, Measured value: 551.4)

**Synthesis Example 9: Synthesis of Intermediate F**

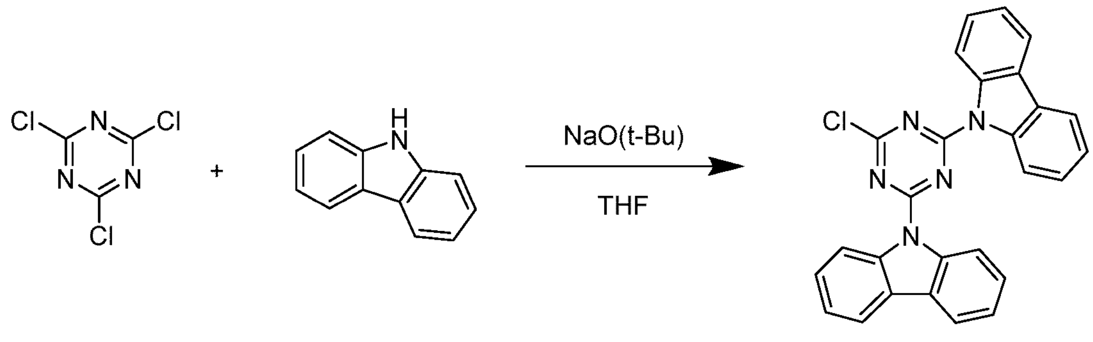


F

10 g (54.23 mmol) of cyanuric chloride and 18.13 g (108.45 mmol) of carbazole were suspended in 250 ml of THF, and 7.81 g (81.35 mmol) of NaO(t-Bu) was slowly added thereto and then, stirred for 12 hours. When a reaction was complete, a solid generated therein was filtered and then, washed with distilled water and acetone/hexane to obtain 12 g (a yield of 49.6 %) of Intermediate F.

**Synthesis Example 10: Synthesis of Comparative Compound R2**

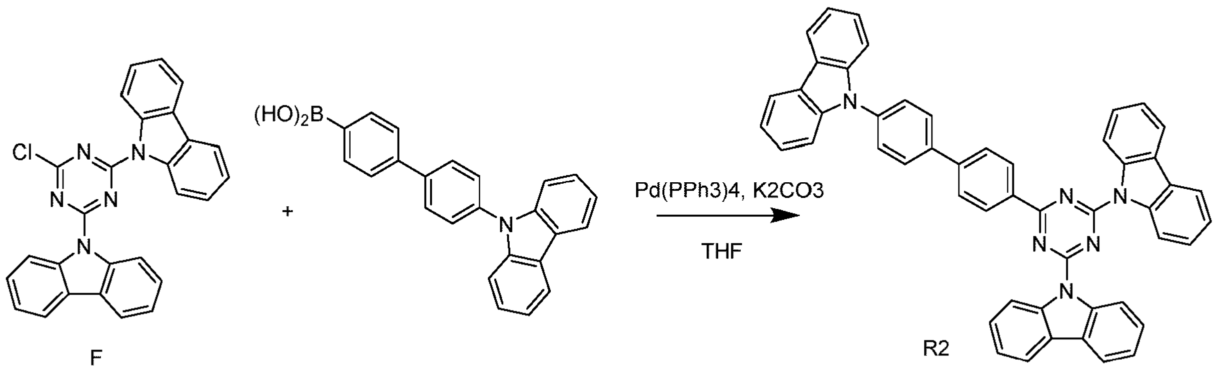


F R2

6 g (13.46 mmol) of Intermediate F synthesized according to Synthesis Example 9, 5.13 g (14.13 mmol) of 4-(9H-carbazole-9-yl)biphenyl boronic acid, 0.47 g (0.40 mmol) of $Pd(PPh_3)_4$, and 3.72 g (26.91 mmol) of $K_2CO_3$ were suspended in 100 ml of THF and 50 ml of distilled water and then, stirred for 12 hours. When a reaction was complete, a solid generated therein was filtered and washed with distilled water/acetone. The solid was heated with 300 ml of dichlorobenzene and dissolved therein and then, silica-gel filtered, a filtrate therefrom was cooled down to room temperature, and a solid generated therein was filtered and dried to obtain 3.9 g (a yield of 40%) of Comparative Compound R2.
(LC/MS : Theoretical value: 728.84, Measured value: 729.6)

**Synthesis Example 11: Synthesis of Intermediate G**

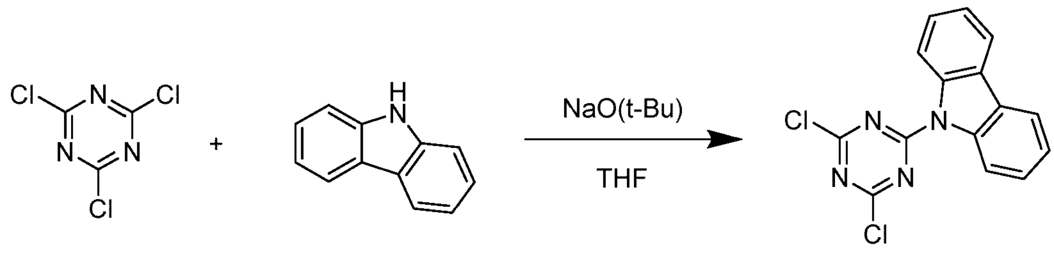


G

36.9 g (200 mmol) of cyanuric chloride and 33.4 g (200 mmol) of carbazole were suspended in 300 ml of THF, and 19. 22 g (200 mmol) of NaO(t-Bu) was slowly added thereto at 0 ° C. When a reaction was complete, a solid generated therein was filtered and then, washed with distilled water/acetone/hexane to obtain 10 g (a yield of 16%) of Intermediate G as a target compound.

**Synthesis Example 12: Synthesis of Intermediate H**

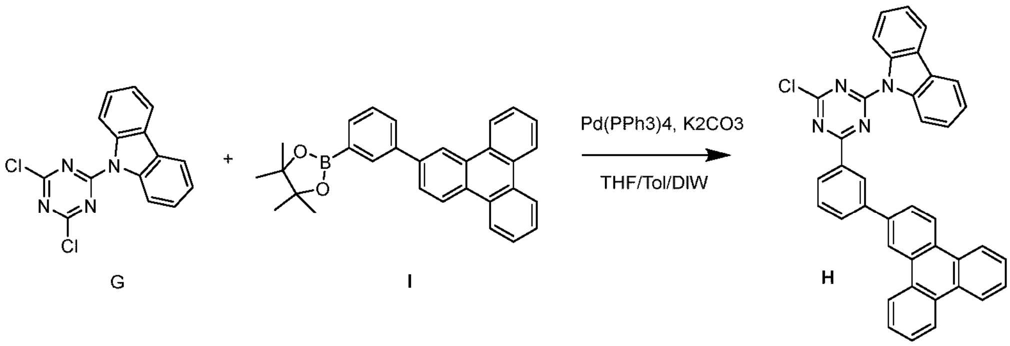


G I H

4.9 g (15.55 mmol) of Intermediate G synthesized according to Synthesis Example 11, 6.69 g (15.55 mmol) of Intermediate I (refer to Synthesis Example 3 of Korea Patent Laid-Open Publication No. 10-2014-0135524), 0.89 g (0.78 mmol) of $Pd(PPh_3)_4$, and 5.37 g (38.87 mmol) of $K_2CO_3$ were suspended in 50 ml of THF/50 ml of toluene/50 ml of distilled water and then, stirred for 12 hours. When a reaction was complete, a solid generated therein was filtered, washed with distilled water/acetone, and dried to obtain 5 g (a yield of 56 %) of Intermediate H as a target compound.

**Synthesis Example 13: Synthesis of Comparative Compound R3**

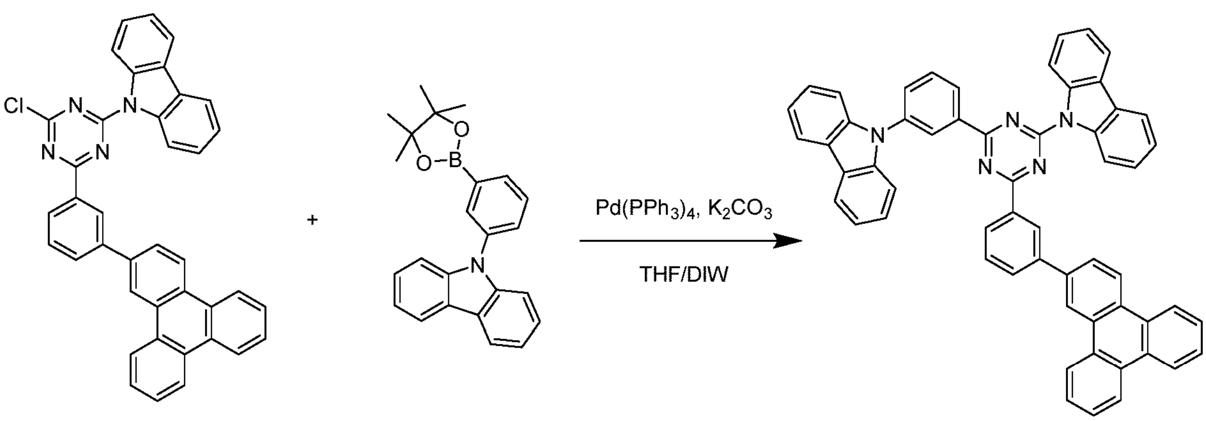


2.4 g (4.11 mmol) of Intermediate H synthesized according to Synthesis Example 12, 1.67 g (4.53 mmol) of 9-[3-(4,4,5,5-tetramethyl-1,3,2-dioxaborolan-2-yl)phenyl]-9H-carbazole, 0.14 g (0.12 mmol) of $Pd(PPh_3)_4$, and 1.14 g

(8.23 mmol) of $K_2CO_3$ were suspended in 50 ml of THF and 50 ml of DIW and then, refluxed and stirred for 12 hours. When a reaction was complete, a solid generated therein was filtered and then, washed with distilled water and acetone. The solid was heated with 100 ml of dichlorobenzene and dissolved therein and then, silica-gel filtered, and a filtrate therefrom was cooled down to room temperature. A solid generated in the filtrate was filtered and washed with acetone to obtain 2.94 g (a yield of 89.5%) of Comparative Compound R3 as a target compound.
(LC/MS : Theoretical value: 789.92, Measured value: 790.40)

**(Preparation of Second Compound for Organic Optoelectronic Device)**

**Synthesis Example 14: Synthesis of Compound B-99**

Compound **B-99** was synthesized in the same manner as known in US2017-0317293A1.

**Synthesis Example 15: Synthesis of Compound C-4**

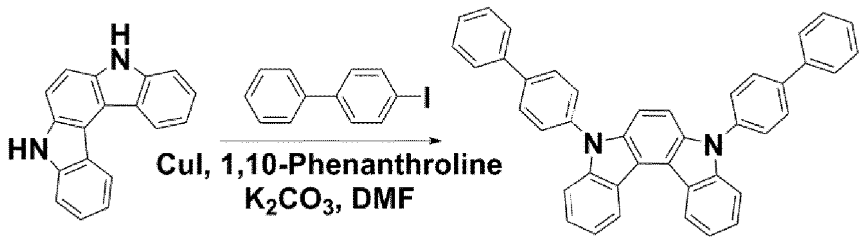


8 g (31.2 mmol) of 5,8-Dihydroindolo[2,3-c]carbazole (CAS No.: 200339-30-6), 20.5 g (73.32 mmol) of 4-iodobiphenyl, 1.19 g (6.24 mmol) of CuI, 1.12 g (6.24 mmol) of 1,10-phenanthoroline, and 12.9 g (93.6 mmol) of $K_2CO_3$ were put in a round-bottomed flask, and 50 ml of DMF was added thereto and then, refluxed and stirred under a nitrogen atmosphere for 24 hours. When a reaction was complete, distilled water was added thereto to extract crystals, which were filtered. The solid was dissolved in 250 ml of xylene and then, silica gel filtered and extracted as a white solid to obtain 16.2 g (a yield of 93 %) of Compound C-4.
LC/MS calculated for: C42H28N2 Exact Mass: 560.2252 found for: 561.23

**(Manufacture of Organic Light Emitting Diode)**

**Example 1**

A glass substrate coated with ITO (indium tin oxide) as a 1500 Å-thick thin film was washed with distilled water. After washing with the distilled water, the glass substrate was ultrasonic wave-washed with a solvent such as isopropyl alcohol, acetone, methanol, and the like and dried and then, moved to a plasma cleaner, cleaned by using oxygen plasma for 10 minutes, and moved to a vacuum depositor. This obtained ITO transparent electrode was used as an anode, Compound A was vacuum-deposited on the ITO substrate to form a 700 Å-thick hole injection layer, and Compound B was deposited to be 50 Å-thick on the injection layer, and then Compound C was deposited to be 1020 Å-thick to form a hole transport layer. On the hole transport layer, 400 Å-thick light emitting layer was formed by using A-1 of Synthesis Example 4 as a host and 7 wt% of PhGD as a dopant by a vacuum-deposition. Subsequently, on the light emitting layer, a 300 Å-thick electron transport layer was formed by simultaneously vacuum-depositing Compound D and Liq in a ratio of 1:1, and on the electron transport layer, Liq and Al were sequentially vacuum-deposited to be 15 Å-thick and 1200 Å-thick, manufacturing an organic light emitting diode.

The organic light emitting diode had a five-layered organic thin layer, and specifically the following structure.

ITO/Compound A (700 Å)/Compound B (50 Å)/Compound C (1020 Å)/EML[A-1:PhGD (7wt%)] (400 Å)/Compound D:Liq (300 Å)/Liq (15 Å)/Al (1200 Å).

Compound A: N4,N4'-diphenyl-N4,N4'-bis(9-phenyl-9H-carbazol-3-yl)biphenyl-4,4'-diamine
Compound B: 1,4,5,8,9,11-hexaazatriphenylene-hexacarbonitrile (HAT-CN)
Compound C: N-(biphenyl-4-yl)-9,9-dimethyl-N-(4-(9-phenyl-9H-carbazol-3-yl)phenyl)-9H-fluoren-2-amine
Compound D: 8-(4-(4,6-di(naphthalen-2-yl)-1,3,5-triazin-2-yl)phenyl)quinoline

[NPB]

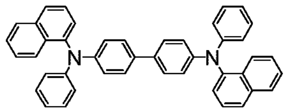

[BAlq]

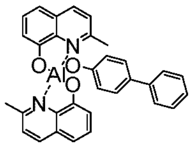

[CBP]

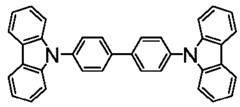

PhGD

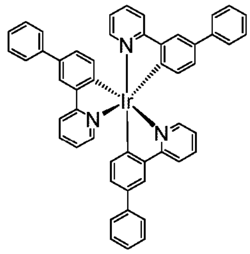

**Examples 2 to 9 and Comparative Examples 1 to 5**

Each diode of Example 2 to Example 9 and Comparative Example 1 to Comparative Example 5 was manufactured according to the same method as Example 1 except that the hosts and ratios thereof were changed as shown in Tables 1 to 5.

**Evaluation: Life-span Increasing Synergy Effect**

Life-span characteristics of the organic light emitting diodes according to Example 1 to Example 9 and Comparative Example 1 to Comparative Example 5 were evaluated. Specific measurement methods are as follows, and the results are shown in Tables 1 to 5.

(1) Measurement of Life-span

T90 life-spans of the organic light emitting diodes according to Examples 1 to 9 and Comparative Examples 1 to 5 were measured as a time when their luminance decreased to 90% relative to the initial luminance (cd/$m^2$) after emitting light with 24000 cd/$m^2$ as the initial luminance (cd/$m^2$) and measuring their luminance decrease depending on a time with a Polanonix life-span measurement system.

(2) Calculation of T90 life-span ratio (%)

Relative comparison values of T90 (h) between a single host or a mixed host examples (first compound as the first host) with the same second host and a mixed host comparative example (comparative compound as the first host).

T90 life-span ratio (%) = {[T90 (h) of Example (first compound used alone or as a mixed host) / [T90 (h) of Comparative Example (comparative compound used alone or as a mixed host)]} X 100

[Table 1]

| | Single host | T90 life-span ratio (%) |
|---|---|---|
| Example 1 | A-1 | 180% |
| Example 2 | A-3 | 150% |
| Comparative Example 1 | R1 | 100% |

[Table 2]

| | Single host | T90 life-span ratio (%) |
|---|---|---|
| Example 3 | A-1 | 350% |
| Example 4 | A-3 | 300% |
| Comparative Example 2 | R2 | 100% |

[Table 3]

| | Single host | T90 life-span ratio (%) |
|---|---|---|
| Example 5 | A-1 | 192% |
| Example 6 | A-3 | 143% |
| Comparative Example 3 | R3 | 100% |

[Table 4]

| | Host | | | T90 life-span ratio (%) |
|---|---|---|---|---|
| | First host | Second host | First and second host ratio | |
| Example 7 | A-1 | B-99 | 3:7 | 150% |
| Example 8 | A-3 | B-99 | 3:7 | 120% |
| Comparative Example 4 | R1 | B-99 | 3:7 | 100% |

[Table 5]

| | Host | | | T90 life-span ratio (%) |
|---|---|---|---|---|
| | First host | Second host | First and second host ratio | |
| Example 9 | A-1 | C-4 | 5:5 | 130% |
| Comparative Example 5 | R1 | C-4 | 5:5 | 100% |

Referring to Tables 1 to 5, the compounds according to the present invention exhibited significantly improved life-spans compared with the comparative compounds.

## Claims

1. A compound for an organic optoelectronic device represented by Chemical Formula 1:

[Chemical Formula 1]

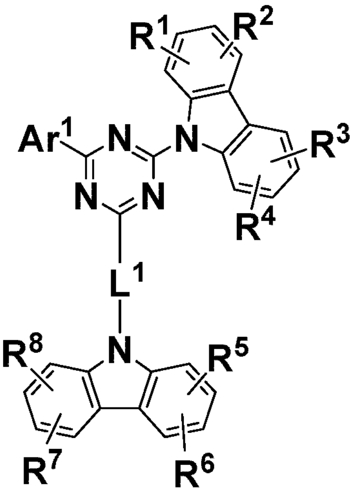

wherein, in Chemical Formula 1,
$Ar^1$ is a substituted or unsubstituted phenyl group or a substituted or unsubstituted biphenyl group,
$L^1$ is an unsubstituted biphenylene group, and
$R^1$ to $R^8$ are independently hydrogen or deuterium.

2. The compound for the organic optoelectronic device of claim 1, wherein $L^1$ is selected from the linking groups of Group I.

[Group Ⅰ]

L-1 L-2 L-3 L-4 L-5 L-6

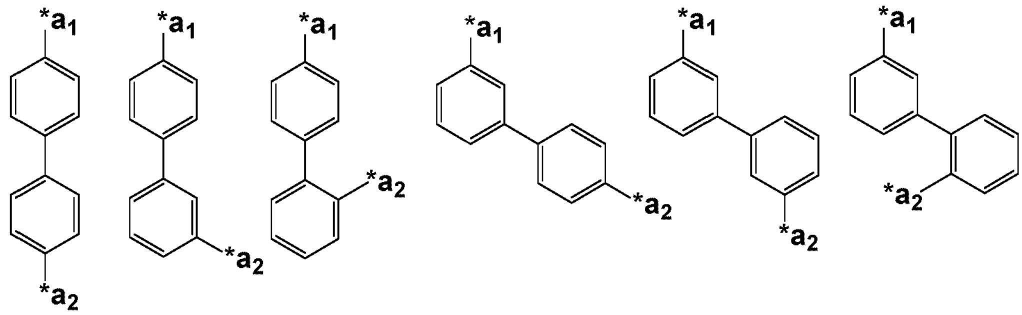

wherein, in Group I , $*a_1$ is a linking portion with triazine of Chemical Formula 1, and $*a_2$ is a linking portion with carbazole of Chemical Formula 1.

3. The compound for the organic optoelectronic device of claim 2, wherein $L^1$ is represented by L-2 or L-4.

4. The compound for the organic optoelectronic device of claim 1, wherein $Ar^1$ is a phenyl group unsubstituted or substituted with deuterium, a phenyl group unsubstituted or substituted with a cyano group, a phenyl group unsubstituted or substituted with a C1 to C5 alkylsilyl group, or an unsubstituted biphenyl group.

5. The compound for the organic optoelectronic device of claim 1, which is a compound of Group 1:

[Group 1]

A-1

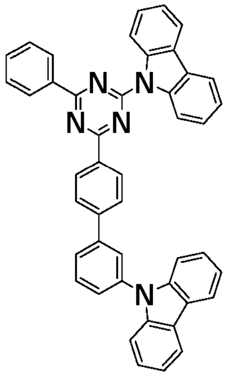

A-2

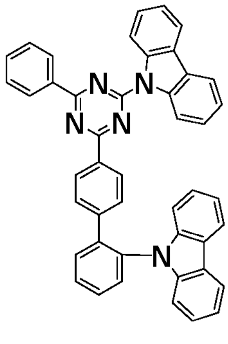

A-3

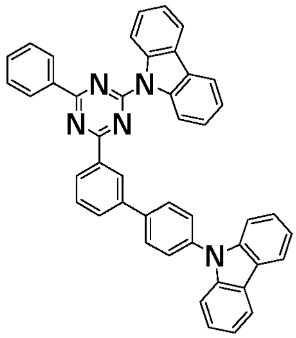

A-4

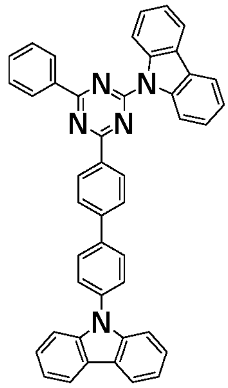

A-5

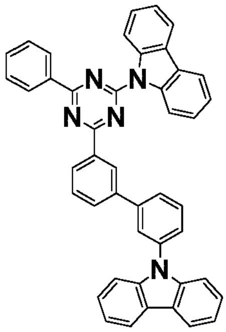

A-6

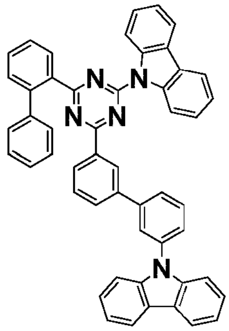

A-7

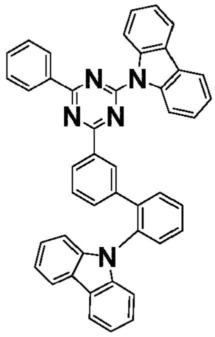

A-8

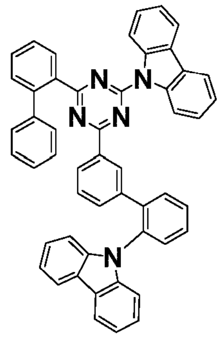

A-9

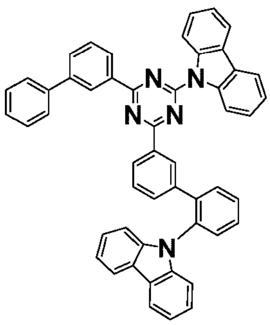

A-10

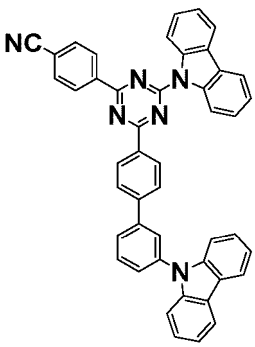

A-11

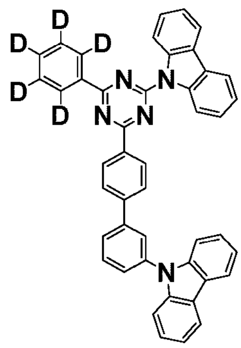

A-12

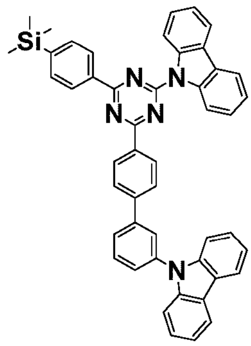

A-13

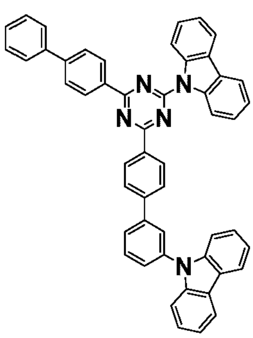

A-14

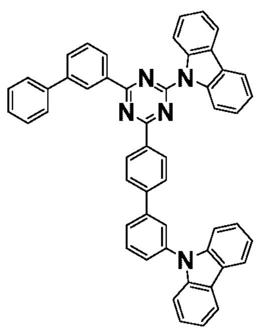

A-15

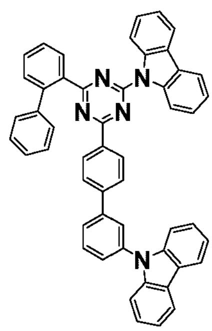

.

**6.** The composition for the organic optoelectronic device, comprising

a first compound for an organic optoelectronic device and a second compound for an organic optoelectronic device,
wherein the first compound for the organic optoelectronic device is represented by Chemical Formula 1 of claim 1, the second compound for the organic optoelectronic device is represented by Chemical Formula 2 or a combination of Chemical Formula 3 and Chemical Formula 4:

[Chemical Formula 2]

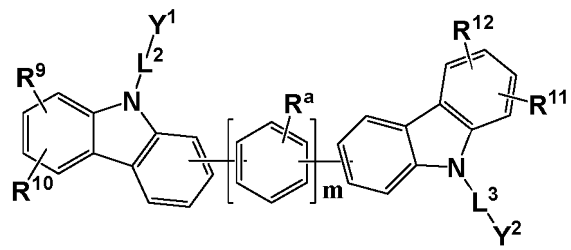

wherein, in Chemical Formula 2,
$Y^1$ and $Y^2$ are independently a substituted or unsubstituted C6 to C20 aryl group or a substituted or unsubstituted C2 to C30 heterocyclic group,
$L^2$ and $L^3$ are independently a single bond, or a substituted or unsubstituted C6 to C20 arylene group,
$R^a$ and $R^9$ to $R^{12}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, and
m is an integer of 0 to 2;

[Chemical Formula 3] [Chemical Formula 4]

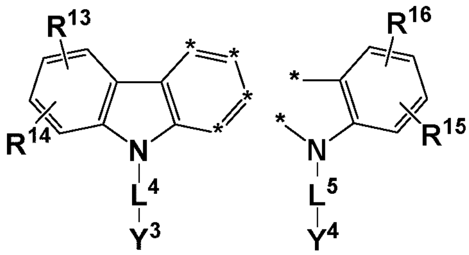

wherein, in Chemical Formula 3 and Chemical Formula 4,
$Y^3$ and $Y^4$ are independently a substituted or unsubstituted C6 to C20 aryl group or a substituted or unsubstituted C2 to C30 heterocyclic group,
adjacent two *'s of Chemical Formula 3 is linked with Chemical Formula 4,
* of Chemical Formula 3 that are not linked with Chemical Formula 4 are independently C-$L^a$-$R^b$,
$L^a$, $L^4$, and $L^5$ are independently a single bond, or a substituted or unsubstituted C6 to C20 arylene group, and
$R^b$ and $R^{13}$ to $R^{16}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

7. The composition for the organic optoelectronic device of claim 6, wherein Chemical Formula 2 is represented by one of Chemical Formula 2-1 to Chemical Formula 2-15:

[Chemical Formula 2-1] [Chemical Formula 2-2] [Chemical Formula 2-3]

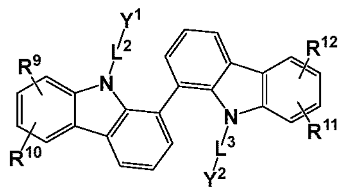 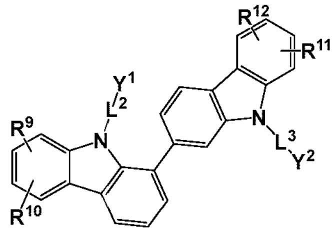 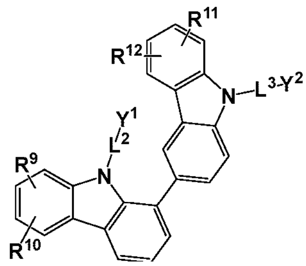

[Chemical Formula 2-4] [Chemical Formula 2-5] [Chemical Formula 2-6]

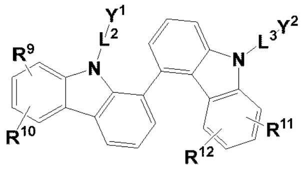 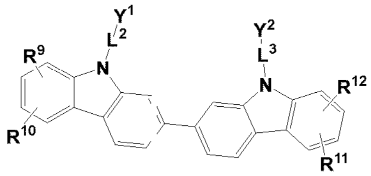 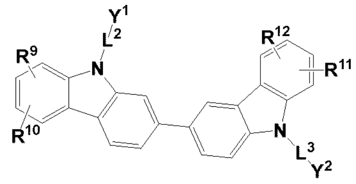

[Chemical Formula 2-7] [Chemical Formula 2-8] [Chemical Formula 2-9]

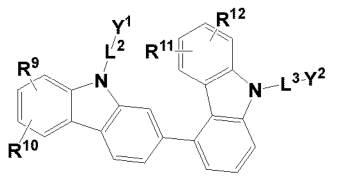 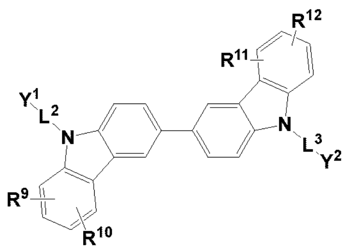 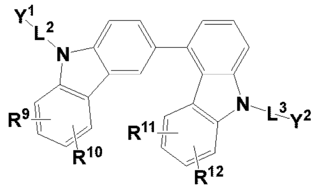

[Chemical Formula 2-10] [Chemical Formula 2-11] [Chemical Formula 2-12]

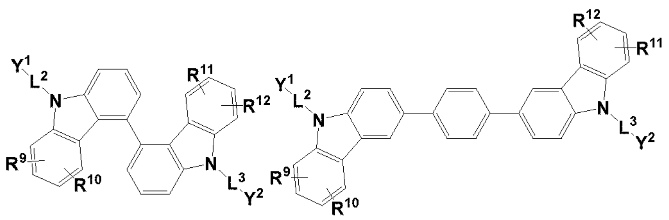 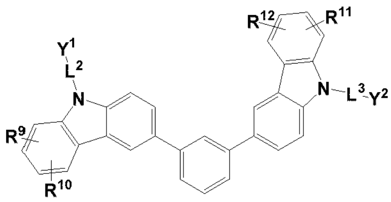

[Chemical Formula 2-13] [Chemical Formula 2-14] [Chemical Formula 2-15]

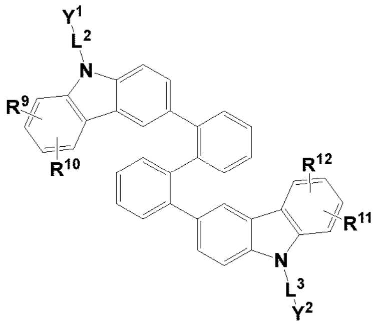

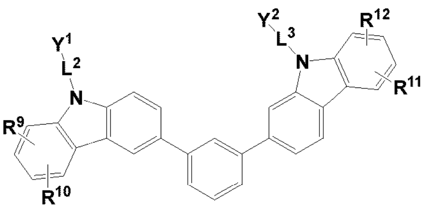

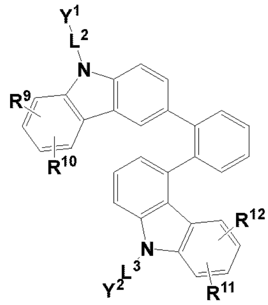

wherein, in Chemical Formula 2-1 to Chemical Formula 2-15,
$R^9$ to $R^{12}$ are independently hydrogen, or a substituted or unsubstituted C6 to C12 aryl group, and
*-$L^2$-$Y^1$ and *-$L^3$-$Y^2$ are independently one of substituents of Group II,

[Group Ⅱ]

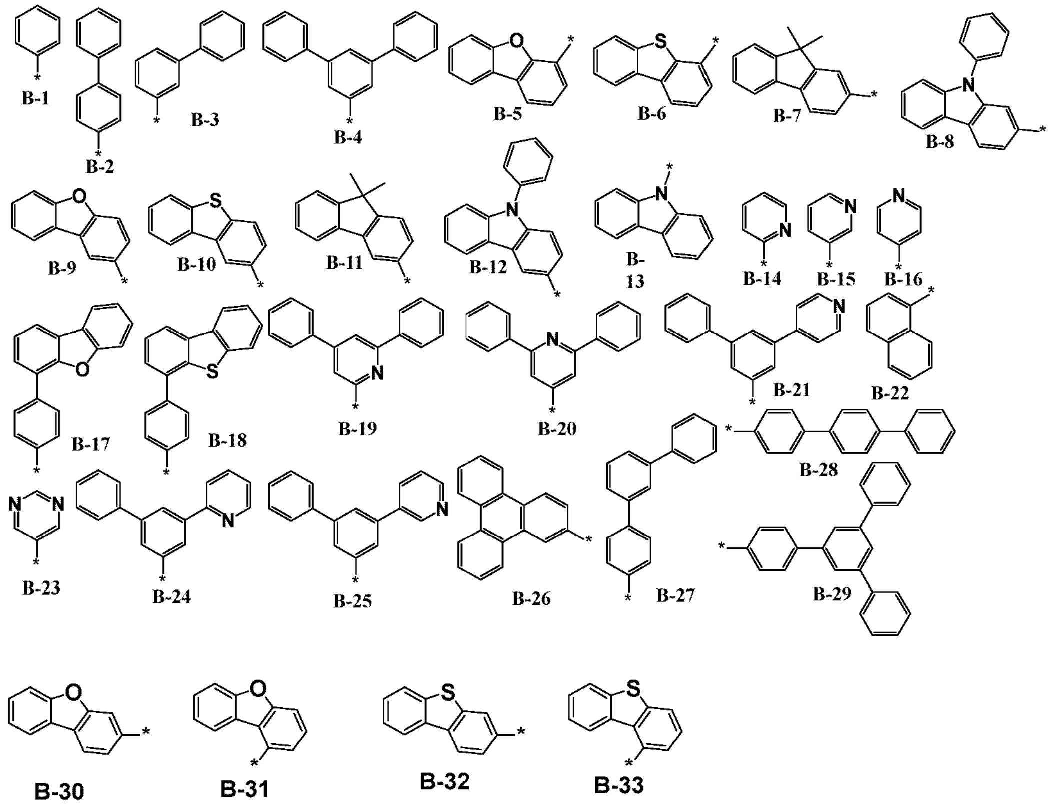

B-1 B-2 B-3 B-4 B-5 B-6 B-7 B-8 B-9 B-10 B-11 B-12 B-13 B-14 B-15 B-16 B-17 B-18 B-19 B-20 B-21 B-22 B-23 B-24 B-25 B-26 B-27 B-28 B-29 B-30 B-31 B-32 B-33

wherein, in Group II, * is a linking portion.

**8.** The composition for the organic optoelectronic device of claim 7, wherein Chemical Formula 2 is represented by Chemical Formula 2-8, and wherein optional *-$L^2$-$Y^1$ and *-$L^3$-$Y^2$ are independently B-1, B-2, and B-3 of Group III.

**9.** The composition for the organic optoelectronic device of claim 6, wherein the combination of Chemical Formula 3 and Chemical Formula 4 is represented by Chemical Formula 3C:

[Chemical Formula 3C]

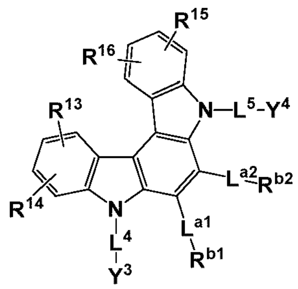

wherein, in Chemical Formula 3C,
$Y^3$ and $Y^4$ are independently a substituted or unsubstituted C6 to C20 aryl group or a substituted or unsubstituted C2 to C30 heterocyclic group,
$L^{a1}$, $L^{a2}$, $L^4$ and $L^5$ are independently a single bond, or a substituted or unsubstituted C6 to C20 arylene group, and
$R^{b1}$, $R^{b2}$ and $R^{13}$ to $R^{16}$ are independently hydrogen, deuterium, a cyano group, a halogen group, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

**10.** The composition for the organic optoelectronic device of claim 9, wherein $Y^3$ and $Y^4$ are independently one of substituents of Group III:

[Group Ⅲ]

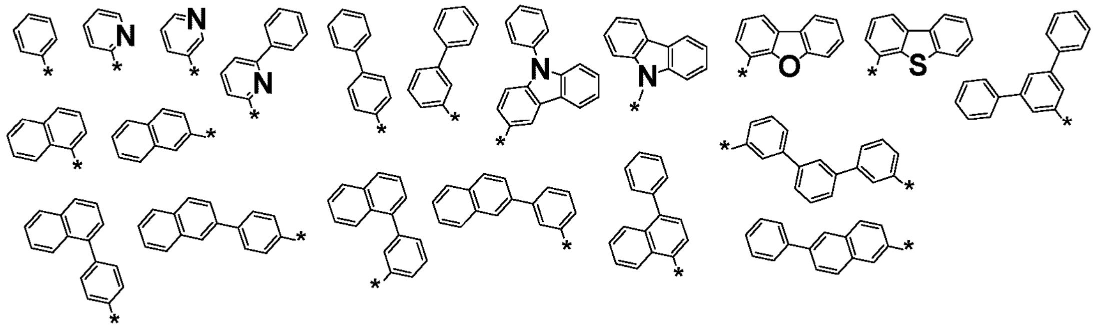

wherein, in Group III,
* is a linking portion with $L^4$ and $L^5$, respectively.

**11.** The composition for the organic optoelectronic device of claim 6, wherein the second compound for the organic optoelectronic device is represented by Chemical Formula 2-8 or Chemical Formula 3C:

[Chemical Formula 2-8] [Chemical Formula 3C]

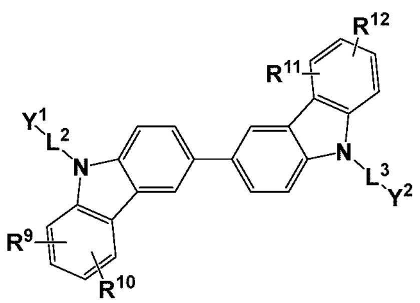 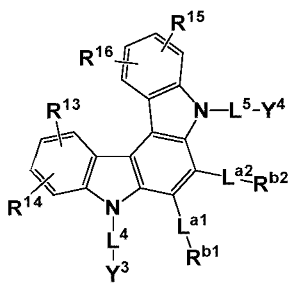

wherein, in Chemical Formula 2-8 and Chemical Formula 3C,

$Y^1$ to $Y^4$ are independently a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group,
$L^2$ to $L^5$, $L^{a1}$, and $L^{a2}$ are independently a single bond, or a substituted or unsubstituted C6 to C20 arylene group, and
$R^{b1}$, $R^{b2}$, and $R^9$ to $R^{16}$ are independently hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

**12.** An organic optoelectronic device, comprising

an anode and a cathode facing each other,
at least one organic layer disposed between the anode and the cathode,
wherein the organic layer comprises the compound for the organic optoelectronic device of any one of claim 1 to claim 6, or
the composition for the organic optoelectronic device of any one of claim 6 to claim 11.

**13.** The organic optoelectronic device of claim 12, wherein

the organic layer comprises a light emitting layer, and
the light emitting layer comprises the compound for the organic optoelectronic device or the composition for the organic optoelectronic device.

**14.** A display device comprising the organic optoelectronic device of claim 12.

**Patentansprüche**

**1.** Verbindung für eine organische optoelektronische Vorrichtung, die durch die Chemische Formel 1 wiedergegeben wird:

[Chemische Formel 1]

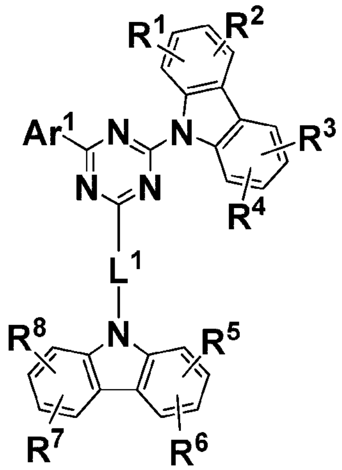

wobei in der Chemischen Formel 1
$Ar^1$ für eine substituierte oder unsubstituierte Phenylgruppe oder eine substituierte oder unsubstituierte Biphenylgruppe steht,
$L^1$ für eine unsubstituierte Biphenylengruppe steht und $R^1$ bis $R^8$ unabhängig für Wasserstoff oder Deuterium stehen.

**2.** Verbindung für die organische optoelektronische Vorrichtung nach Anspruch 1, wobei $L^1$ aus den Verknüpfungsgruppen der Gruppe I ausgewählt ist:

[Gruppe I]

L-1 L-2 L-3 L-4 L-5 L-6

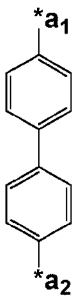
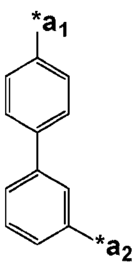
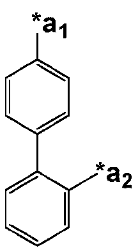
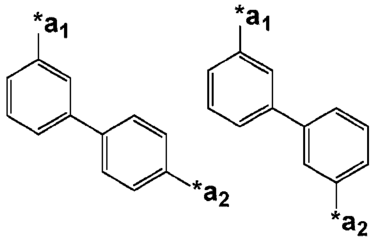
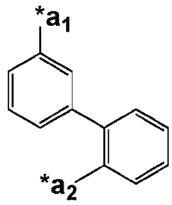

wobei in Gruppe I $*a_1$ für einen Verknüpfungsteil mit dem Triazin der Chemischen Formel 1 steht und $*a_2$ für einen Verknüpfungsteil mit dem Carbazol der Chemischen Formel 1 steht.

**3.** Verbindung für die organische optoelektronische Vorrichtung nach Anspruch 2, wobei $L^1$ durch L-2 oder L-4 wiedergegeben wird.

**4.** Verbindung für die organische optoelektronische Vorrichtung nach Anspruch 1, wobei $Ar^1$ für eine Phenylgruppe, die unsubstituiert oder durch Deuterium substituiert ist, eine Phenylgruppe, die unsubstituiert oder durch eine Cyanogruppe substituiert ist, eine Phenylgruppe, die unsubstituiert oder durch eine C1- bis C5- Alkylsilylgruppe substituiert ist, oder eine unsubstituierte Biphenylgruppe steht.

**5.** Verbindung für die organische optoelektronische Vorrichtung nach Anspruch 1, bei der es sich um eine Verbindung der Gruppe 1 handelt:

[Gruppe 1]

A-1

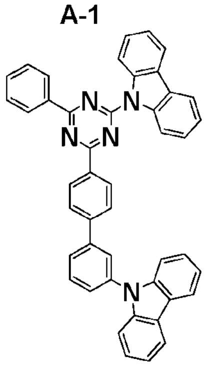

A-2

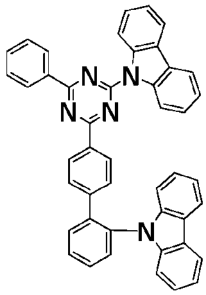

A-3

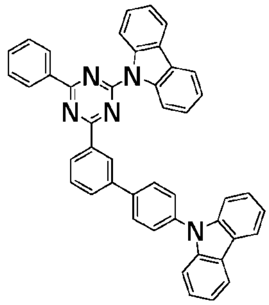

A-4

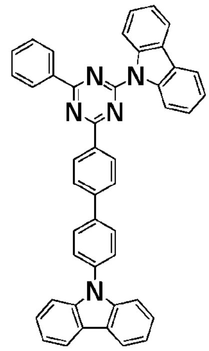

A-5

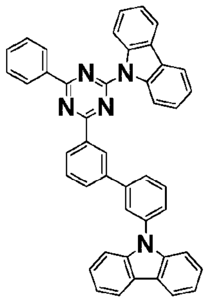

A-6

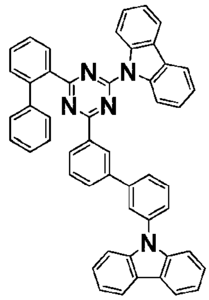

A-7

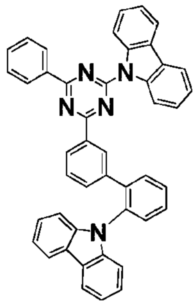

A-8

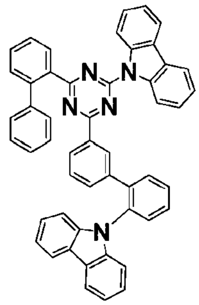

A-9

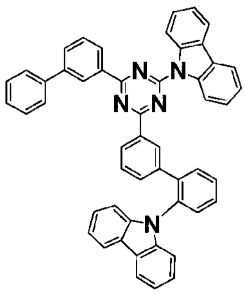

A-10

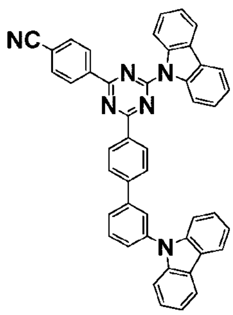

A-11

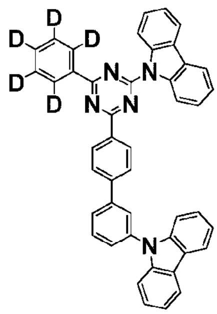

A-12

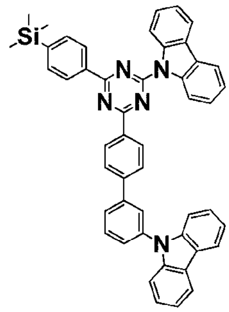

A-13

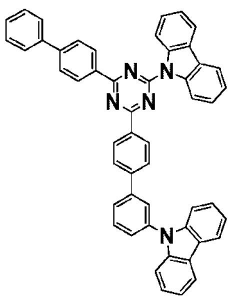

A-14

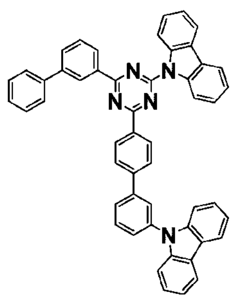

A-15

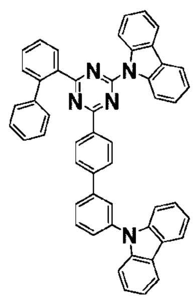

**6.** Zusammensetzung für die organische optoelektronische Vorrichtung, umfassend eine erste Verbindung für eine organische optoelektronische Vorrichtung und eine zweite Verbindung für eine organische optoelektronische Vorrichtung, wobei die erste Verbindung für die organische optoelektronische Vorrichtung durch die Chemische Formel 1 nach Anspruch 1 wiedergegeben wird,

die zweite Verbindung für die organische optoelektronische Vorrichtung durch die Chemische Formel 2 oder eine Kombination der Chemischen Formel 3 und der Chemischen Formel 4 wiedergegeben wird:

[Chemische Formel 2]

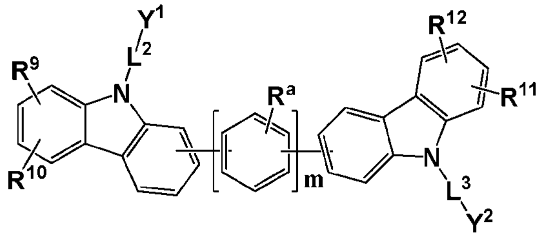

wobei in der Chemischen Formel 2
$Y^1$ und $Y^2$ unabhängig für eine substituierte oder unsubstituierte C6- bis C20-Arylgruppe oder eine substituierte oder unsubstituierte heterocyclische C2-bis C30-Gruppe stehen,
$L^2$ und $L^3$ unabhängig für eine Einfachbindung oder eine substituierte oder unsubstituierte C6 bis C20-Arylengruppe stehen,
$R^a$ und $R^9$ bis $R^{12}$ unabhängig für Wasserstoff, Deuterium, eine Cyanogruppe, eine Halogengruppe, eine substituierte oder unsubstituierte Amingruppe, eine substituierte oder unsubstituierte C1- bis C30-Alkylgruppe, eine substituierte oder unsubstituierte C6- bis C30-Arylgruppe, eine substituierte oder unsubstituierte heterocyclische C2- bis C30-Gruppe stehen und
m für eine ganze Zahl von 0 bis 2 steht;

[Chemische Formel 3] [Chemische Formel 4]

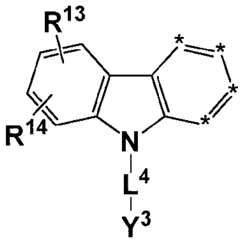

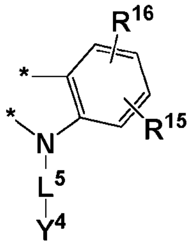

wobei in der Chemischen Formel 3 und der Chemischen Formel 4
$Y^3$ und $Y^4$ unabhängig für eine substituierte oder unsubstituierte C6- bis C20-Arylgruppe oder eine substituierte oder unsubstituierte heterocyclische C2-bis C30-Gruppe stehen,
benachbarte zwei * der Chemischen Formel 3 mit der Chemischen Formel 4 verknüpft sind,
* der Chemischen Formel 3, die nicht mit der Chemischen Formel 4 verknüpft sind, unabhängig für C-$L^a$-$R^b$ stehen, $L^a$, $L^4$ und $L^5$ unabhängig für eine Einfachbindung oder eine substituierte oder unsubstituierte C6- bis C20-Arylengruppe stehen und
$R^b$ und $R^{13}$ bis $R^{16}$ unabhängig für Wasserstoff, Deuterium, eine Cyanogruppe, eine Halogengruppe, eine substituierte oder unsubstituierte Amingruppe, eine substituierte oder unsubstituierte C1- bis C30-Alkylgruppe, eine substituierte oder unsubstituierte C6- bis C30-Arylgruppe oder eine substituierte oder unsubstituierte heterocyclische C2- bis C30-Gruppe stehen.

**7.** Zusammensetzung für die organische optoelektronische Vorrichtung nach Anspruch 6, wobei die Chemische Formel 2 durch eine der Chemischen Formeln 2-1 bis 2-15 wiedergegeben wird:

[Chemische Formel 2-1] [Chemische Formel 2-2] [Chemische Formel 2-3]

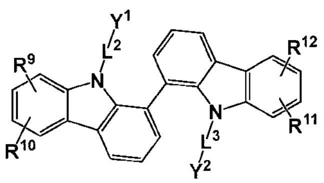

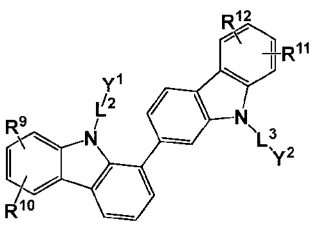

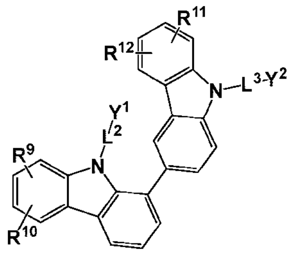

[Chemische Formel 2-4] [Chemische Formel 2-5] [Chemische Formel 2-6]

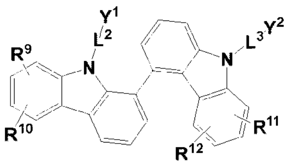
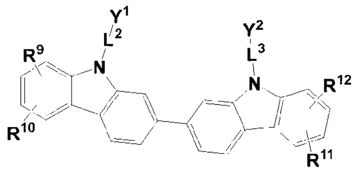
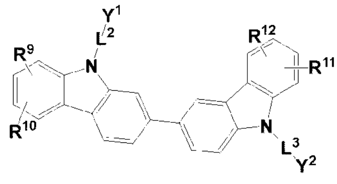

[Chemische Formel 2-7] [Chemische Formel 2-8] [Chemische Formel 2-9]

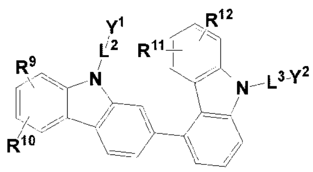
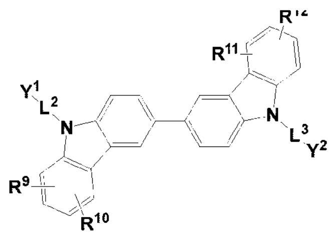
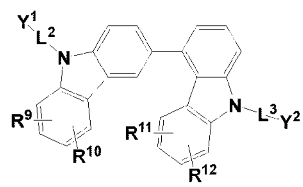

[Chemische Formel 2-10] [Chemische Formel 2-11] [Chemische Formel 2-12]

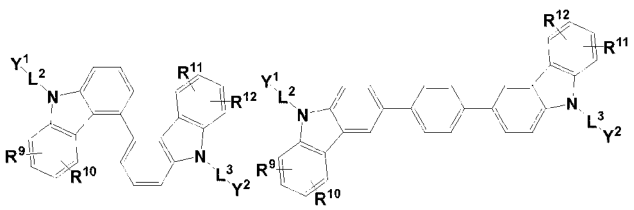
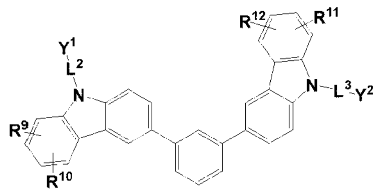

[Chemische Formel 2-13] [Chemische Formel 2-14] [Chemische Formel 2-15]

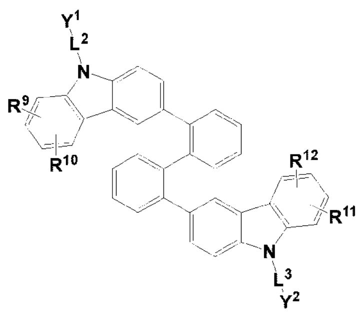
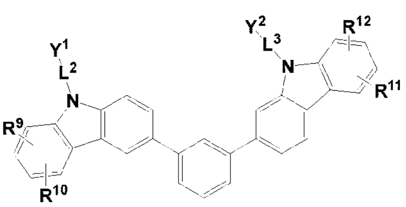
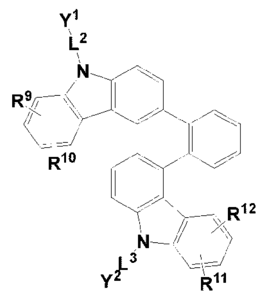

wobei in den Chemischen Formeln 2-1 bis 2-15
$R^9$ bis $R^{12}$ unabhängig für Wasserstoff oder eine substituierte oder unsubstituierte C6- bis C12-Arylgruppe stehen und
*-$L^2$-$Y^1$ und *-$L^3$-$Y^2$ unabhängig für einen der Substituenten der Gruppe II stehen,

[Gruppe II]

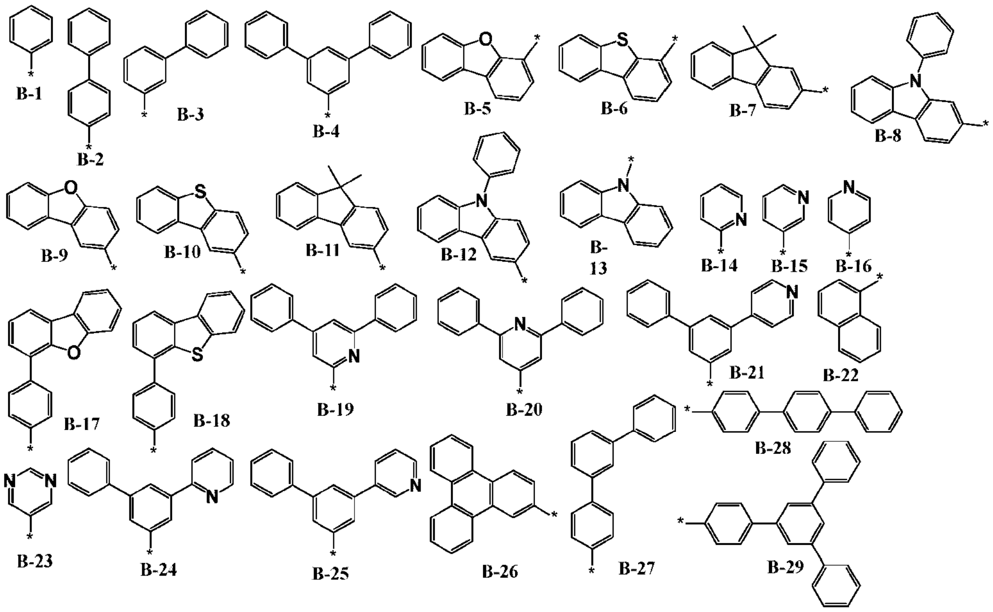

B-1 B-2 B-3 B-4 B-5 B-6 B-7 B-8 B-9 B-10 B-11 B-12 B-13 B-14 B-15 B-16 B-17 B-18 B-19 B-20 B-21 B-22 B-23 B-24 B-25 B-26 B-27 B-28 B-29

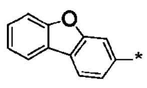

**B-30**

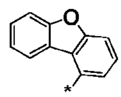

**B-31**

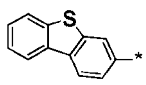

**B-32**

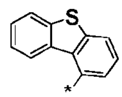

**B-33**

wobei in Gruppe II * für einen Verknüpfungsteil steht.

**8.** Zusammensetzung für die organische optoelektronische Vorrichtung nach Anspruch 7, wobei die Chemische Formel 2 durch die Chemische Formel 2-8 wiedergegeben wird und wobei gegebenenfalls $*-L^2-Y^1$ und $*-L^3-Y^2$ unabhängig für B-1, B-2 und B-3 der Gruppe III stehen.

**9.** Zusammensetzung für die organische optoelektronische Vorrichtung nach Anspruch 6, wobei die Kombination der Chemischen Formel 3 und der Chemischen Formel 4 durch die Chemische Formel 3C wiedergegeben wird:

[Chemische Formel 3C]

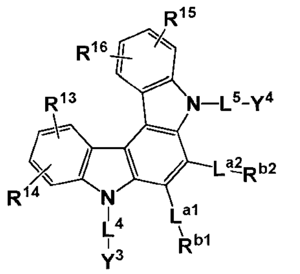

wobei in der Chemischen Formel 3C
$Y^3$ und $Y^4$ unabhängig für eine substituierte oder unsubstituierte C6- bis C20-Arylgruppe oder eine substituierte oder unsubstituierte heterocyclische C2-bis C30-Gruppe stehen,
$L^{a1}$, $L^{a2}$, $L^4$ und $L^5$ unabhängig für eine Einfachbindung oder eine substituierte oder unsubstituierte C6- bis C20-Arylengruppe stehen und
$R^{b1}$, $R^{b2}$ und $R^{13}$ bis $R^{16}$ unabhängig für Wasserstoff, Deuterium, eine Cyanogruppe, eine Halogengruppe, eine substituierte oder unsubstituierte Amingruppe, eine substituierte oder unsubstituierte C1- bis C30-Alkylgruppe, eine substituierte oder unsubstituierte C6-bis C30-Arylgruppe oder eine substituierte oder unsubstituierte heterocyclische C2- bis C30-Gruppe stehen.

**10.** Zusammensetzung für die organische optoelektronische Vorrichtung nach Anspruch 9, wobei $Y^3$ und $Y^4$ unabhängig für einen der Substituenten der Gruppe III stehen:

[Gruppe III]

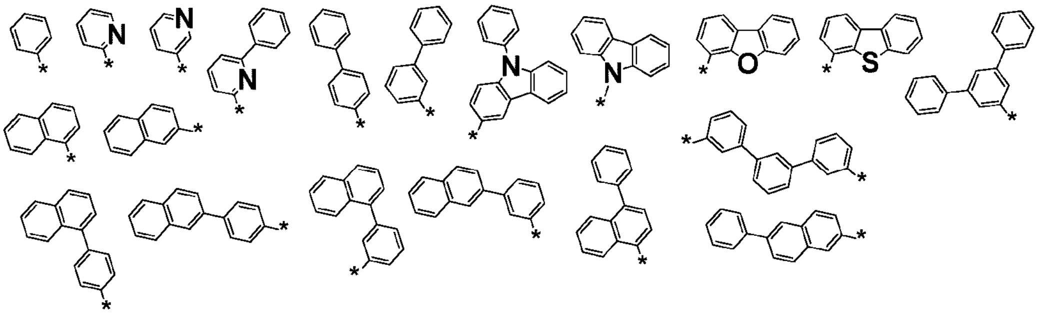

wobei in Gruppe III
* für einen Verknüpfungsteil mit $L^4$ bzw. $L^5$ steht.

**11.** Zusammensetzung für die organische optoelektronische Vorrichtung nach Anspruch 6, wobei die zweite Verbindung für eine organische optoelektronische Vorrichtung durch die Chemische Formel 2-8 oder die Chemische Formel 3C wiedergegeben wird:

[Chemische Formel 2-8] [Chemische Formel 3C]

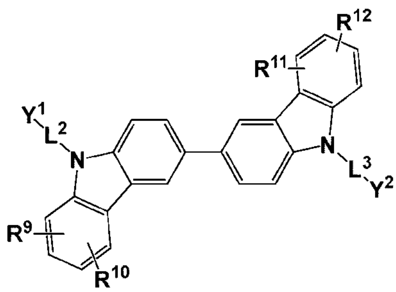

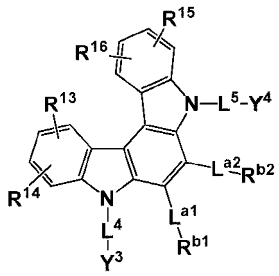

wobei in der Chemischen Formel 2-8 und der Chemischen Formel 3C
$Y^1$ bis $Y^4$ unabhängig für eine substituierte oder unsubstituierte Phenylgruppe, eine substituierte oder unsubstituierte Biphenylgruppe, eine substituierte oder unsubstituierte Pyridinylgruppe, eine substituierte oder unsubstituierte Carbazolylgruppe, eine substituierte oder unsubstituierte Dibenzofuranylgruppe oder eine substituierte oder unsubstituierte Dibenzothiophenylgruppe stehen,
$L^2$ bis $L^5$, $L^{a1}$ und $L^{a2}$ unabhängig für eine Einfachbindung oder eine substituierte oder unsubstituierte C6- bis C20-Arylengruppe stehen und
$R^{b1}$, $R^{b2}$ und $R^9$ bis $R^{16}$ unabhängig für Wasserstoff, Deuterium, eine Cyanogruppe, eine substituierte oder unsubstituierte Phenylgruppe, eine substituierte oder unsubstituierte Biphenylgruppe, eine substituierte oder unsubstituierte Pyridinylgruppe, eine substituierte oder unsubstituierte Carbazolylgruppe, eine substituierte oder unsubstituierte Dibenzofuranylgruppe oder eine substituierte oder unsubstituierte Dibenzothiophenylgruppe stehen.

**12.** Organische optoelektronische Vorrichtung, umfassend eine Anode und eine Kathode, die einander zugewandt sind, mindestens eine organische Schicht, die zwischen der Anode und der Kathode angeordnet ist,

wobei die organische Schicht die Verbindung für die organische optoelektronische Vorrichtung nach einem der Ansprüche 1 bis 6 oder
die Zusammensetzung für die organische optoelektronische Vorrichtung nach einem der Ansprüche 6 bis 11 umfasst.

**13.** Organische optoelektronische Vorrichtung nach Anspruch 12, wobei

die organische Schicht eine lichtemittierende Schicht umfasst und
die lichtemittierende Schicht die Verbindung für die organische optoelektronische Vorrichtung oder die Zusammensetzung für die organische optoelektronische Vorrichtung umfasst.

**14.** Anzeigevorrichtung, umfassend die organische optoelektronische Vorrichtung nach Anspruch 12.

## Revendications

**1.** Composé pour un dispositif optoélectronique organique représenté par la formule chimique 1 :

[Formule chimique 1]

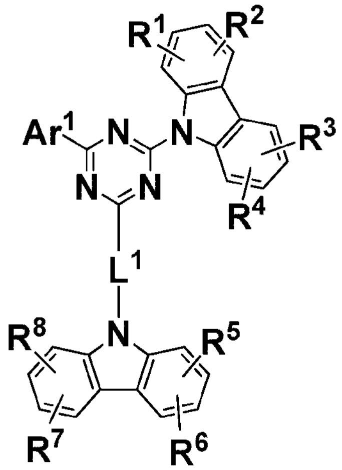

dans laquelle, dans la formule chimique 1,
$Ar^1$ est un groupe phényle substitué ou non substitué ou un groupe biphényle substitué ou non substitué,
$L^1$ est un groupe biphénylène non substitué, et
$R^1$ à $R^8$ sont indépendamment un hydrogène ou du deutérium.

**2.** Composé pour le dispositif optoélectronique organique selon la revendication 1, dans lequel $L^1$ est choisi parmi les groupes de liaison du groupe I.

[Groupe I]

L-1 L-2 L-3 L-4 L-5 L-6

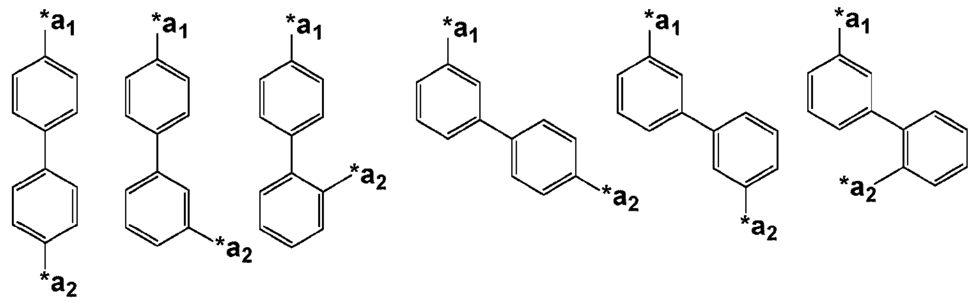

dans lequel, dans le groupe I, $*a_1$ est une partie de liaison avec la triazine de formule chimique 1, et $*a_2$ est une partie de liaison avec le carbazole de formule chimique 1.

**3.** Composé pour le dispositif optoélectronique organique selon la revendication 2, dans lequel $L^1$ est représenté par L-2 ou L-4.

**4.** Composé pour le dispositif optoélectronique organique selon la revendication 1, dans lequel $Ar^1$ est un groupe phényle non substitué ou substitué par deutérium, un groupe phényle non substitué ou substitué par un groupe cyano,

un groupe phényle non substitué ou substitué par un groupe alkylsilyle C1 à C5, ou un groupe biphényle non substitué.

**5.** Composé pour le dispositif optoélectronique organique selon la revendication 1, qui est un composé du groupe 1 :

[Groupe 1]

**A-1**

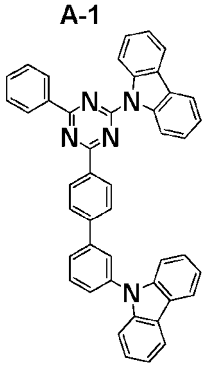

**A-2**

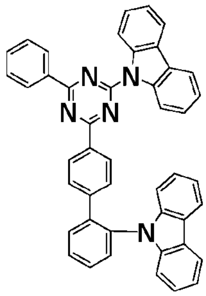

**A-3**

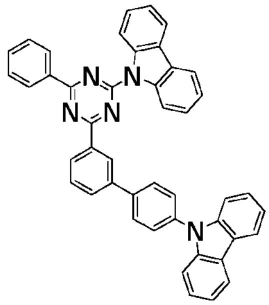

**A-4**

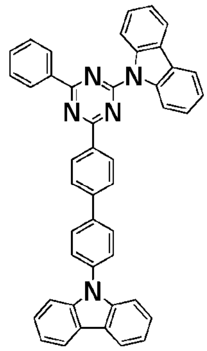

**A-5**

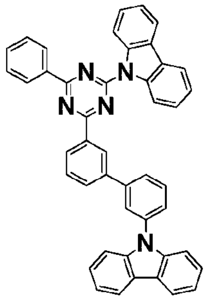

**A-6**

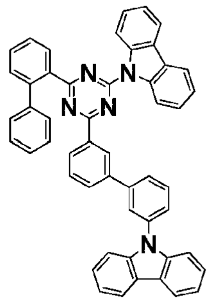

**A-7**

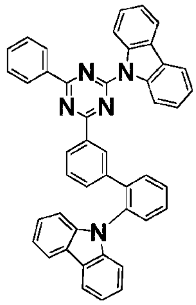

**A-8**

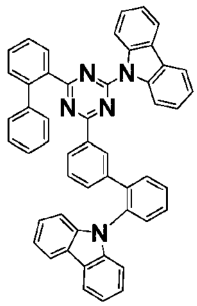

**A-9**

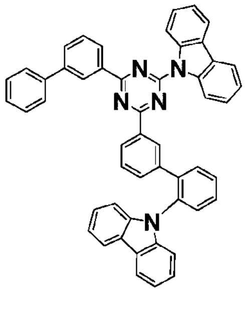

**A-10**

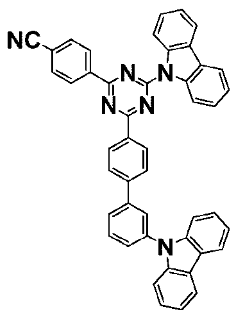

**A-11**

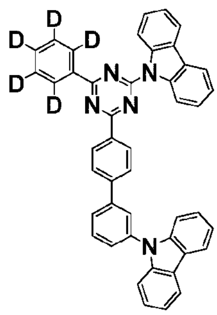

**A-12**

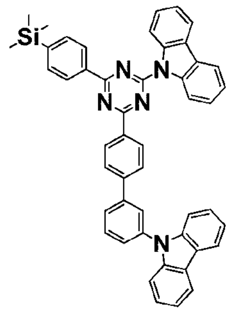

**A-13**

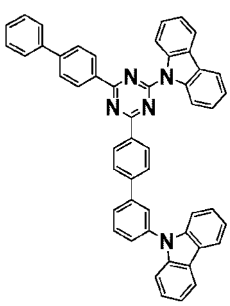

**A-14**

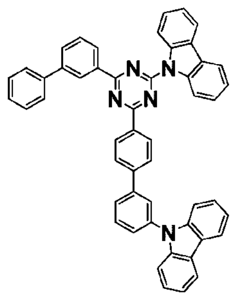

**A-15**

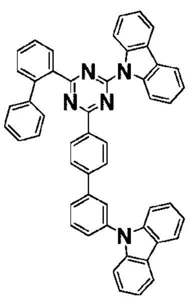

.

**6.** Composition pour le dispositif optoélectronique organique, comprenant

un premier composé pour un dispositif optoélectronique organique et un deuxième composé pour un dispositif optoélectronique organique,

dans laquelle le premier composé pour le dispositif optoélectronique organique est représenté par la formule chimique 1 selon la revendication 1,

le deuxième composé pour le dispositif optoélectronique organique est représenté par la formule chimique 2 ou une combinaison de formule chimique 3 et de formule chimique 4 :

[Formule chimique 2]

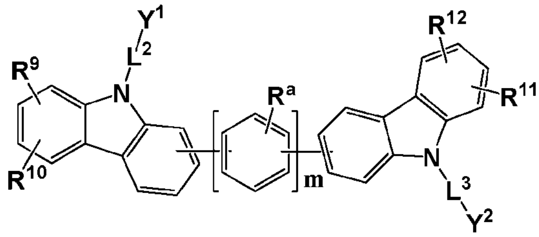

dans laquelle, dans la formule chimique 2,

$Y^1$ et $Y^2$ sont indépendamment un groupe aryle C6 à C20 substitué ou non substitué ou un groupe hétérocyclique C2 à C30 substitué ou non substitué,

$L^2$ et $L^3$ sont indépendamment une simple liaison, ou un groupe arylène C6 à C20 substitué ou non substitué,

$R^a$ et $R^9$ à $R^{12}$ sont indépendamment hydrogène, deutérium, un groupe cyano, un groupe halogène, un groupe amine substitué ou non substitué, un groupe alkyle C1 à C30 substitué ou non substitué, un groupe aryle C6 à C30 substitué ou non substitué, un groupe hétérocyclique C2 à C30 substitué ou non substitué, et

m est un entier de 0 à 2 ;

[Formule chimique 3] [Formule chimique 4]

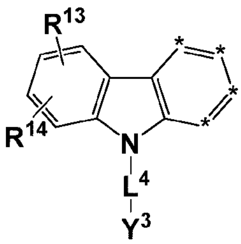

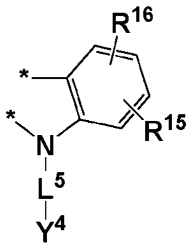

dans laquelle, dans la formule chimique 3 et la formule chimique 4,

$Y^3$ et $Y^4$ sont indépendamment un groupe aryle C6 à C20 substitué ou non substitué ou un groupe hétérocyclique C2 à C30 substitué ou non substitué,

deux * adjacents de la formule chimique 3 sont liés à la formule chimique 4,

* de la formule chimique 3 qui ne sont pas liés à la formule chimique 4 sont indépendamment C-$L^a$-$R^b$,

$L^a$, $L^4$, et $L^5$ sont indépendamment une simple liaison, ou un groupe arylène C6 à C20 substitué ou non substitué, et

$R^b$ et $R^{13}$ à $R^{16}$ sont indépendamment hydrogène, deutérium, un groupe cyano, un groupe halogène, un groupe amine substitué ou non substitué, un groupe alkyle C1 à C30 substitué ou non substitué, un groupe aryle C6 à C30 substitué ou non substitué, ou un groupe hétérocyclique C2 à C30 substitué ou non substitué

7. Composition pour le dispositif optoélectronique organique selon la revendication 6, dans laquelle la formule chimique 2 est représentée par l'une parmi la formule chimique 2-1 à la formule chimique 2-15 :

[Formule chimique 2-1] [Formule chimique 2-2] [Formule chimique 2-3]

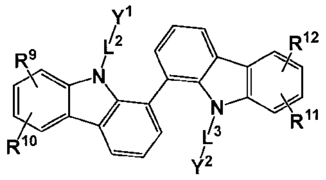
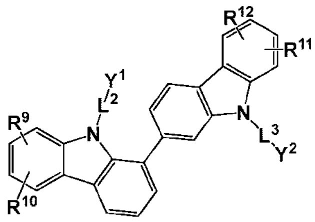
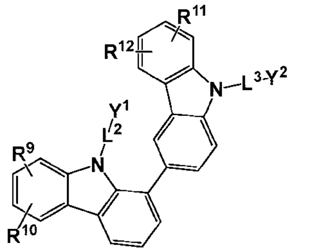

[Formule chimique 2-4] [Formule chimique 2-5] [Formule chimique 2-6]

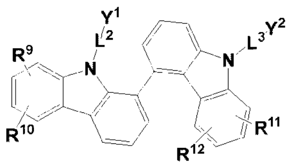
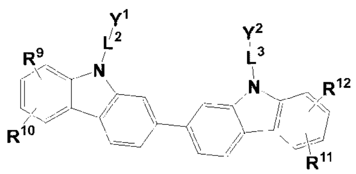
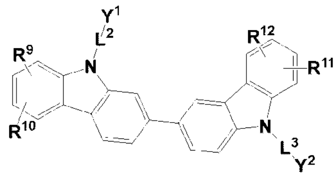

[Formule chimique 2-7] [Formule chimique 2-8] [Formule chimique 2-9]

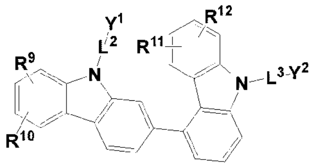
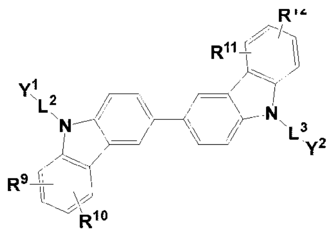
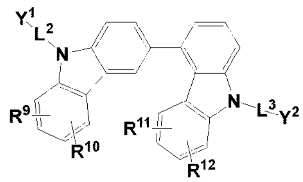

[Formule chimique 2-10] [Formule chimique 2-11] [Formule chimique 2-12]

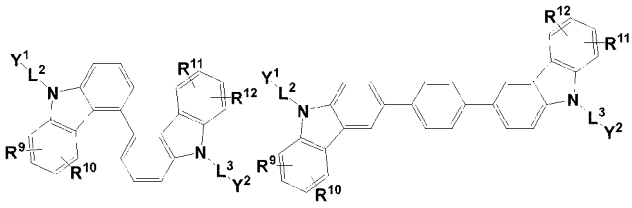
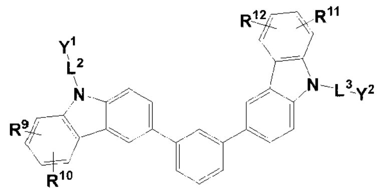

[Formule chimique 2-13] [Formule chimique 2-14] [Formule chimique 2-15]

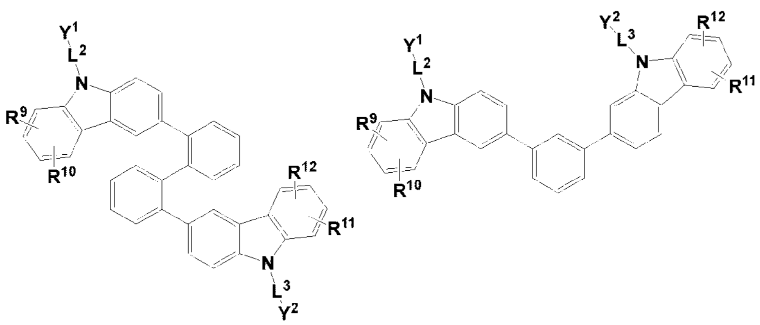
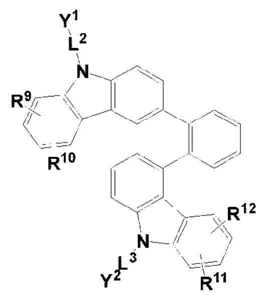

dans laquelle, dans la formule chimique 2-1 à la formule chimique 2-15,
$R^9$ à $R^{12}$ sont indépendamment hydrogène, ou un groupe aryle C6 à C12 substitué ou non substitué, et
$*-L^2-Y^1$ et $*-L^3-Y^2$ sont indépendamment l'un des substituants du groupe II,

[Groupe II]

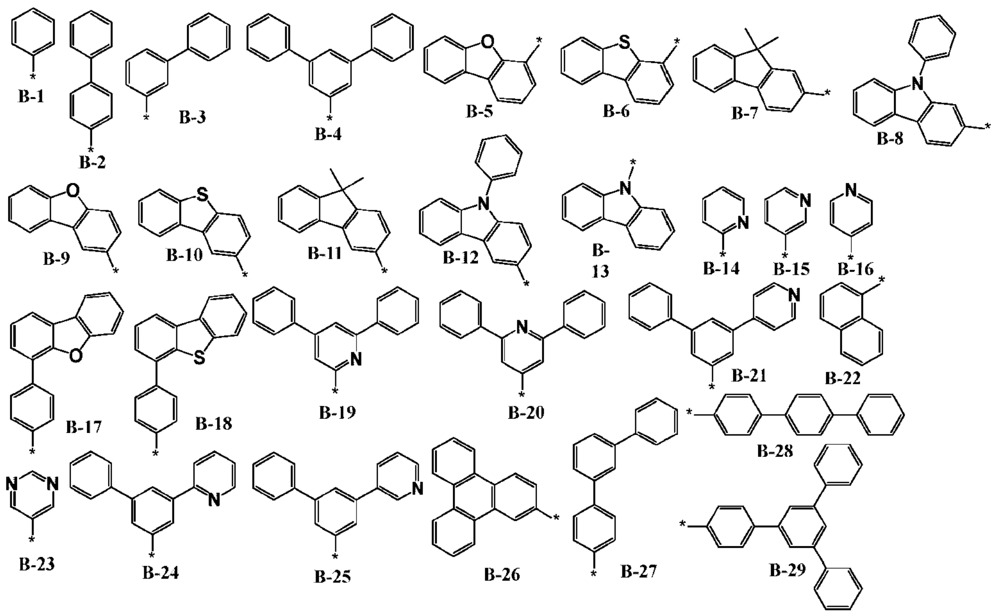

B-1 B-2 B-3 B-4 B-5 B-6 B-7 B-8 B-9 B-10 B-11 B-12 B-13 B-14 B-15 B-16 B-17 B-18 B-19 B-20 B-21 B-22 B-23 B-24 B-25 B-26 B-27 B-28 B-29

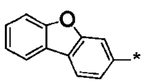

B-30

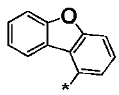

B-31

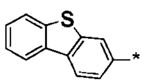

B-32

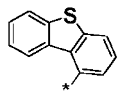

B-33

dans laquelle, dans le groupe II, * est une partie de liaison.

**8.** Composition pour le dispositif optoélectronique organique selon la revendication 7, dans laquelle la formule chimique 2 est représentée par la formule chimique 2-8, et dans laquelle $*-L^2-Y^1$ et $*-L^3-Y^2$ éventuels sont indépendamment B-1, B-2, et B-3 du groupe III.

**9.** Composition pour le dispositif optoélectronique organique selon la revendication 6, dans laquelle la combinaison de formule chimique 3 et de formule chimique 4 est représentée par la formule chimique 3C :

[Formule chimique 3C]

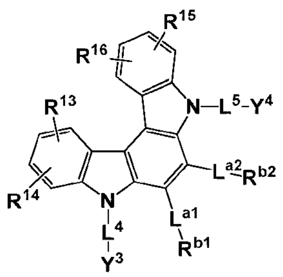

dans laquelle, dans la formule chimique 3C,
$Y^3$ et $Y^4$ sont indépendamment un groupe aryle C6 à C20 substitué ou non substitué ou un groupe hétérocyclique C2 à C30 substitué ou non substitué,
$L^{a1}$, $L^{a2}$, $L^4$ et $L^5$ sont indépendamment une simple liaison, ou un groupe arylène C6 à C20 substitué ou non substitué, et
$R^{b1}$, $R^{b2}$ et $R^{13}$ à $R^{16}$ sont indépendamment hydrogène, deutérium, un groupe cyano, un groupe halogène, un

groupe amine substitué ou non substitué, un groupe alkyle C1 à C30 substitué ou non substitué, un groupe aryle C6 à C30 substitué ou non substitué, ou un groupe hétérocyclique C2 à C30 substitué ou non substitué.

**10.** Composition pour le dispositif optoélectronique organique selon la revendication 9, dans laquelle $Y^3$ et $Y^4$ sont indépendamment l'un des substituants du groupe III :

[Groupe III]

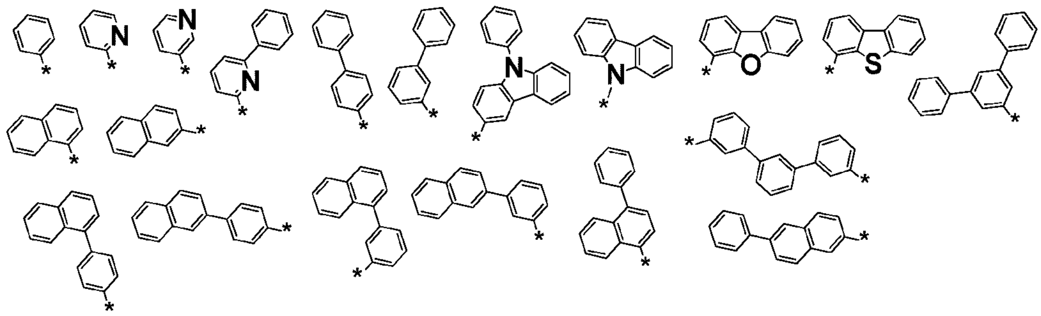

dans laquelle, dans le groupe III,
* est une partie de liaison avec $L^4$ et $L^5$ respectivement.

**11.** Composition pour le dispositif optoélectronique organique selon la revendication 6, dans laquelle le deuxième composé pour le dispositif optoélectronique organique est représenté par la formule chimique 2-8 ou la formule chimique 3C :

[Formule chimique 2-8] [Formule chimique 3C]

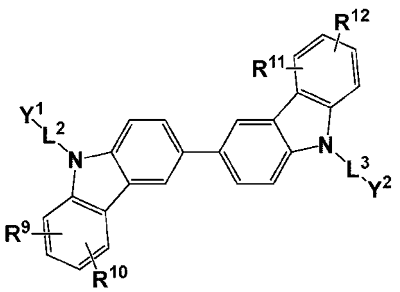

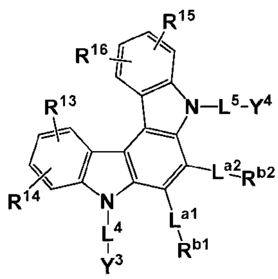

dans laquelle, dans la formule chimique 2-8 et la formule chimique 3C,
$Y^1$ à $Y^4$ sont indépendamment un groupe phényle substitué ou non substitué, un groupe biphényle substitué ou non substitué, un groupe pyridinyle substitué ou non substitué, un groupe carbazolyle substitué ou non substitué, un groupe dibenzofuranyle substitué ou non substitué, ou un groupe dibenzothiophényle substitué ou non substitué,
$L^2$ à $L^5$, $L^{a1}$, et $L^{a2}$ sont indépendamment une simple liaison, ou un groupe arylène C6 à C20 substitué ou non substitué, et
$R^{b1}$, $R^{b2}$, et $R^9$ à $R^{16}$ sont indépendamment hydrogène, deutérium, un groupe cyano, un groupe phényle substitué ou non substitué, un groupe biphényle substitué ou non substitué, un groupe pyridinyle substitué ou non substitué, un groupe carbazolyle substitué ou non substitué, un groupe dibenzofuranyle substitué ou non substitué, ou un groupe dibenzothiophényle substitué ou non substitué.

**12.** Dispositif optoélectronique organique, comprenant une anode et une cathode se faisant face,

au moins une couche organique disposée entre l'anode et la cathode,
dans lequel la couche organique comprend le composé pour le dispositif organique optoélectronique selon l'une quelconque de la revendication 1 à la revendication 6 ; ou
la composition pour le dispositif optoélectronique organique selon l'une quelconque de la revendication 6 à la revendication 11.

**13.** Dispositif optoélectronique organique selon la revendication 12, dans lequel

la couche organique comprend une couche émettrice de lumière, et
la couche émettrice de lumière comprend le composé pour le dispositif optoélectronique organique ou la composition pour le dispositif optoélectronique organique.

**14.** Dispositif d'affichage comprenant le dispositif optoélectronique organique selon la revendication 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103435597 A
- US 5061569 A
- JP 1993 A
- JP 009471 A
- WO 1995009147 A1
- JP 7126615 A
- JP 10095973 A
- KR 1020140135524
- US 20170317293 A1


### Non-patent literature cited in the description


- **DANIEL WAGNER et al.** Triazine Based Bipolar Hast Materials for Blue Phosphorescent OLEDs. *Chemistry of materials*, 13 August 2013, vol. 25 (18), ISSN 0897-4756, 3758-3765
- *CHEMICAL ABSTRACTS*, 200339-30-6